# EUROPEAN PATENT APPLICATION

(11) **EP 4 692 372 A1**
(43) Date of publication of application: **11.02.2026**
(21) Application number: 24306350.0
(22) Date of filing: 09.08.2024
(51) Int. Cl.: C12Q 1/6876, C12N 15/66, C12N 15/79

(54) **UNIVERSAL LANDING PLATFORM**

(71) Applicant: UNIVERCELLS FRANCE SAS, 91080 Evry-Courcouronnes (FR)
(72) Inventor: RAHIER, Renaud, 91080 Evry-Courcouronnes (FR); CHARBONNIER, Teddy, 91080 Evry- Courcouronnes (FR); SAID, Ahmed, 91000 Evry-Courcouronnes (FR); RANDRIANJATOVO-GBALOU, Irina, 10190 Bucey-en-Othe (FR)
(74) Representative: Icosa

(57) **Abstract**

The present invention relates to a DNA molecule comprising a nucleic acid sequence comprising at least 3 restriction enzymes sites, preferably at least 4 restriction enzymes sites, as well as vectors or cells comprising thereof.

## Description

### FIELD OF INVENTION

The present invention relates to products, in particular deoxyribonucleic acid molecules, comprising multiple restriction enzymes sites, and vectors, in particular expression vectors comprising the same.

The present invention also relates to uses and methods preparing, and/or for replicating nucleic acids.

The present invention also relates to the field of RNA-based drugs and in particular messenger RNA ("mRNA")-based drugs.

### BACKGROUND OF INVENTION

mRNA-based drugs are powerful and versatile tools for treating genetic diseases, cancer and infectious diseases (*e.g*., mRNA vaccines). For the manufacture of mRNA vaccines, there is thus a need to produce large quantities of nucleic acids which may be administered in the form of mature or pre-mature mRNAs.

Such applications generally require a first step of cloning into plasmids/vectors, and replication. Accordingly, there is a general need for plasmids/vectors which are compatible with nucleic acid replication and production at an industrial scale; in particular those for preparing RNA molecules, and especially messenger RNAs.

This is generally done *in vitro* with In Vitro Transcription (IVT). IVT requires DNA compatible with the IVT process. For that, the DNA molecule (typically a plasmid bearing a region to be transcribed into a messenger RNA of interest) should be designed in a manner where the messenger RNA is itself capable to retain its function, or alternatively to be prepared for further modification in a manner which is compatible with protein expression; in particular in eukaryotic cells.

Most notably, mature messenger RNAs suitable for eukaryotic translation possess specific characteristics, notably they possess a polyA tail along with additional characteristics which are meant to modulate (e.g. improve) protein production or half time within the cell.

Accordingly, there is a general need in the Art to develop nucleic acid molecules, in particular those comprising a GOI and a poly (dA/dT) tail, which can be efficiently replicated, amplified and produced.

Alternatively, there is a general need in the Art to ensure that such nucleic acid molecules, in particular DNA molecules, can be edited in a targeted manner before or after amplification. Hence, there is a general need for means adapted to nucleic acid replication, amplification, and production.

Targeted editing of DNA molecules can be achieved, for example, by restriction enzyme digestion. To do this, a type II restriction enzyme is often used. The problem is that this class of restriction enzyme must not cut the GOI.

To ensure that the enzyme does not cut on the GOI, or any other DNA molecule containing a sequence of interest, a new enzyme should thus be selected for each new nucleic acid construct, which implies, for example for mRNA production, that a new plasmid should be built for each new GOI. This increases drastically the time and cost of production.

Therefore, there is a need for a "universal" and flexible tool that enables nucleic acid amplification and modification, and which can be used for messenger RNA production.

There is also a need for editing nucleic acids in a reliable manner, at one or more specific sites without disrupting the one or more element(s) essential to its function.

In particular, and in the context of mRNA production, there is a need for plasmids/vectors which are compatible with gene editing, and which are capable of editing one region while preserving the other.

For example, there is a need for editing a plasmid/vector comprising a GOI, or any other region of interest without editing the said GOI and without having to adapt the plasmid/vector or the GOI sequence for each edition.

There is thus a general need for methods for editing, preparing, and/or for replicating nucleic acids, in particular those which are suitable for RNA production and more particularly for messenger RNA production that remain compatible with *in vivo* cell expression.

### SUMMARY OF THE INVENTION

The present invention relates to a deoxyribonucleic acid (DNA) molecule, or composition thereof, comprising at least one nucleic acid sequence of formula (I), or **SEQ ID NO 1:**
(I) **X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆**; wherein:
X₁ is G;
X₂ is selected from a group consisting of: A, C or G;
X₃ is selected from a group consisting of: A, G or T;
X₄ is selected from a group consisting of: A, C, G or T;
X₅ is selected from a group consisting of: A, C or T;
X₆ is selected from a group consisting of: A, C, G or T;
X₇ is selected from a group consisting of: C or G;
X₈ is selected from a group consisting of: A or G;
X₉ is selected from a group consisting of: G or T;
X₁₀ is selected from a group consisting of: A, C, G or T;
X₁₁ is selected from a group consisting of: A, C or T;
X₁₂ is selected from a group consisting of: C, G or T;
X₁₃ is C;
X₁₄ is selected from a group consisting of: A, G or T;
X₁₅ is selected from a group consisting of: A or C;
X₁₆ is C;
X₁₇ is T;
X₁₈ is selected from a group consisting of: C, G or T;
X₁₉ is selected from a group consisting of: A or C;
X₂₀ is selected from a group consisting of: A or G;
X₂₁ is selected from a group consisting of: C, G or T;
X₂₂ is selected from a group consisting of: C or T;
X₂₃ is selected from a group consisting of: C or G;
X₂₄ is G;
X₂₅ is selected from a group consisting of: A or G;
X26 is C.

In some embodiments, the DNA molecule or composition thereof comprises GVDNHNSRKNHBCDMCTBMRBYSGRC (**SEQ ID NO: 1**); R being A or G; Y being C or T; M being A or C; K being G or T; S being C or G; W being A or T; B being C, G or T; D being A, G or T; H being A, C or T; V being A, C or G; N being A, C, G or T; wherein the DNA molecule comprises at least 50 nucleotides.

In some embodiments, the DNA molecule or composition thereof comprises a plurality of restriction enzyme sites selected from type II restriction enzyme sites, preferably type IIC, type IIS and/or type IIP restriction enzyme sites, more preferably selected from the group consisting of: AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PleI, SapI, and SfaNI restriction sites.

In some embodiments, the DNA molecule or composition thereof comprises a plurality of restriction enzyme sites selected from the group consisting of: BmrI, BpuEI, BsaI, and EciI restriction sites.

In some embodiments, the restriction enzymes corresponding to said plurality of restriction enzyme sites cleave the DNA molecule on the same locus.

In some embodiments, the DNA molecule or composition thereof comprises at least one nucleic acid sequence selected from the group consisting of: **SEQ ID NO: 2** or **SEQ ID NO: 3.**

In some embodiments, the DNA molecule or composition thereof comprises at least one nucleic acid sequence selected from the group consisting of: **SEQ ID NO: 5** to **SEQ ID NO: 57.**

In some embodiments, the DNA molecule or composition thereof comprises at least one nucleic acid sequence **SEQ ID NO: 4.**

In some embodiments, the DNA molecule or composition thereof comprises at least two nucleic acid sequences of formula (I), or **SEQ ID NO: 1,** which may be the same or different.

In some embodiments, the DNA molecule or composition thereof comprises a poly(dA/dT) nucleic acid sequence; wherein the poly(dA/dT) nucleic acid sequence is characterized by a nucleic acid length from 10 nucleotides to 150 nucleotides.

In some embodiments, the DNA molecule or composition thereof further comprises:
- at least one 5' Untranslated Region (5' UTR) and at least one 3' Untranslated Region (3' UTR);
- at least one nucleic acid sequence of interest; and
- a poly(dA/dT) nucleic acid sequence.

In some embodiments, the at least one nucleic acid sequence of formula (I), or **SEQ ID NO: 1,** is localized in 5' of said at least one nucleic acid sequence of interest, and at least one nucleic acid sequence of formula (I), or **SEQ ID NO: 1,** is localized in 3' of said poly(dA/dT) nucleic acid sequence.

In some embodiments, the DNA molecule is a circular DNA molecule such as a plasmid, a linear DNA molecule, or a concatemer DNA molecule.

The present invention further relates to method of in vitro transcription, comprising the steps of:
(i) contacting at least one DNA molecule, or composition thereof, according to the invention with at least one RNA polymerase, and
(ii) performing in vitro transcription of said DNA molecule, thereby providing at least one RNA molecule, in particular at least one messenger RNA (mRNA), for example a self-amplifying messenger RNA.

The present invention further relates to a recombinant host cell or microorganism or viral particle comprising the DNA molecule, or composition thereof, according to the invention.

### GENERAL DESCRIPTION OF THE INVENTION

The inventors provide herein a solution with a nucleic acid molecule, in particular a DNA molecule, comprising a sequence comprising multiple restriction enzymes sites.

In particular, the inventors provide herein a "universal landing platform" sequence, or ULP, which comprises a plurality of nucleic acid sequences recognized by type II restriction enzymes, but which are designed to be digested at the same location.

The universal landing platform, which may advantageously comprise three or more type II restriction sites, is thus defined herein as a nucleic acid consensus sequence region including a plurality of wobble bases, consisting of a 26-base or base pair sequence of formula (I), or **SEQ ID NO 1.**

The consensus region is further capable of allowing 53 particular configurations, reported herein as sequences **SEQ ID NO 4 to 57;** encompassed by at least two particular consensus regions **SEQ ID NO 2** and **SEQ ID NO 3.**

This invention thus relates to a nucleic acid molecule, in particular deoxyribonucleic acid (DNA) molecule, or composition thereof, comprising at least one nucleic acid sequence that comprises at least 3, preferably at least 4, restriction enzyme sites.

It will be understood that such nucleic acid molecule, in particular such DNA molecule, enables the use of multiple (3, 4 or 5) different restriction enzymes for cleaving the DNA molecule, preferably at the same site; when the DNA molecule comprises a sequence of interest (e.g., a transgene), it is possible to select a restriction enzyme that cleaves the DNA molecule without cleaving the sequence of interest.

Advantageously, this general consensus region is designed in order to comprise a plurality of restriction sites, most preferably recognized by type II restriction sites, including a selection of enzymes (e.g. BsaI, EciI, BpuEI, and BmrI) for which the recognized cleavage site is found to be statistically under-represented in reference genomic sequences, and which remains compatible with nucleic acid sequences comprising poly (dA/dT) sequences, or with nucleic acid sequences into which a poly (dA/dT) sequence must be inserted or produced (e.g. through replication).

Also advantageously, such ULPs are compatible with standardized digestion protocols, while also serving as a framework for amplification primers compatible with any sequence of interest (e.g. any GOI) that has a poly (dA/dT) "tail" nucleic acid sequence.

While the invention itself relates to the consensus region sequence *per se,* corresponding nucleic acid molecules and compositions thereof, the inventive concept further extends toward nucleic acid constructs comprising one or more additional nucleic acid sequences in 5' or 3' of the ULP. In particular, the invention further relates to nucleic acid molecules, comprising one or more ULP consensus sequence regions, including in particular, and in a non-exhaustive manner:
- nucleic acid molecules, in particular DNA molecules, comprising at least one ULP consensus sequence region and at least one further nucleic acid sequence (e.g. a coding sequence in combination with a poly A/T sequence) of interest in 5' and/or 3' of the ULP; **and/or**
- nucleic acid molecules, in particular DNA molecules, comprising at least one nucleic acid sequence of interest, flanked by at least two ULPs.

Advantageously, the nucleic acid sequence of interest may be further characterized in that it comprises or consists of **(i)** a coding sequence, or CDS, and **(ii)** a poly(dA/dT) nucleic acid sequence.

This invention thus relates to a deoxyribonucleic acid (DNA) molecule, or composition thereof, comprising at least one nucleic acid sequence of formula (I), or **SEQ ID NO 1:**
(I) **X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆**; wherein:
X₁ is G;
X₂ is selected from a group consisting of: A, C or G;
X₃ is selected from a group consisting of: A, G or T;
X₄ is selected from a group consisting of: A, C, G or T;
X₅ is selected from a group consisting of: A, C or T;
X₆ is selected from a group consisting of: A, C, G or T;
X₇ is selected from a group consisting of: C or G;
X₈ is selected from a group consisting of: A or G;
X₉ is selected from a group consisting of: G or T;
X₁₀ is selected from a group consisting of: A, C, G or T;
X₁₁ is selected from a group consisting of: A, C or T;
X₁₂ is selected from a group consisting of: C, G or T;
X₁₃ is C;
X₁₄ is selected from a group consisting of: A, G or T;
X₁₅ is selected from a group consisting of: A or C;
X₁₆ is C;
X₁₇ is T;
X₁₈ is selected from a group consisting of: C, G or T;
X₁₉ is selected from a group consisting of: A or C;
X₂₀ is selected from a group consisting of: A or G;
X₂₁ is selected from a group consisting of: C, G or T;
X₂₂ is selected from a group consisting of: C or T;
X₂₃ is selected from a group consisting of: C or G;
X24 is G;
X₂₅ is selected from a group consisting of: A or G;
X26 is C.

In some embodiments, the DNA molecule or composition thereof comprises GVDNHNSRKNHBCDMCTBMRBYSGRC (**SEQ ID NO: 1**); R being A or G; Y being C or T; M being A or C; K being G or T; S being C or G; W being A or T; B being C, G or T; D being A, G or T; H being A, C or T; V being A, C or G; N being A, C, G or T; optionally wherein the DNA molecule comprises at least 50, at least 500, or at least 5000 nucleotides.

In some embodiments, the DNA molecule or composition thereof comprises a plurality of restriction enzyme sites selected from type II restriction enzyme sites, preferably type IIC, type IIS and/or type IIP restriction enzyme sites, more preferably selected from the group consisting of: AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PleI, SapI, and SfaNI restriction sites.

In some embodiments, the DNA molecule or composition thereof comprises a plurality of restriction enzyme sites selected from the group consisting of: BmrI, BpuEI, BsaI, and EciI restriction sites.

In some embodiments, the restriction enzymes corresponding to the plurality of restriction enzyme sites cleave the DNA molecule on the same locus.

In some embodiments, the DNA molecule or composition thereof comprises at least one nucleic acid sequence selected from the group consisting of: **SEQ ID NO: 2** or **SEQ ID NO: 3.**

In some embodiments, the DNA molecule or composition thereof comprises at least one nucleic acid sequence selected from the group consisting of: **SEQ ID NO: 5** to **SEQ ID NO: 57.**

In some embodiments, the DNA molecule or composition thereof comprises at least one nucleic acid sequence **SEQ ID NO: 4.**

In some embodiments, the DNA molecule or composition thereof comprises at least two nucleic acid sequences of formula (I), or **SEQ ID NO: 1,** which may be the same or different.

In some embodiments, the DNA molecule or composition thereof comprises a poly(dA/dT) nucleic acid sequence; wherein the poly(dA/dT) nucleic acid sequence is characterized by a nucleic acid length from 10 nucleotides to 150 nucleotides.

In some embodiments, the DNA molecule or composition thereof further comprises:
- at least one 5' Untranslated Region (5' UTR) and at least one 3' Untranslated Region (3' UTR);
- at least one nucleic acid sequence of interest; and
- a poly(dA/dT) nucleic acid sequence.

In some embodiments, the at least one nucleic acid sequence of formula (I), or **SEQ ID NO: 1,** is localized in 5' of the at least one nucleic acid sequence of interest, and at least one nucleic acid sequence of formula (I), or **SEQ ID NO: 1,** is localized in 3' of the poly(dA/dT) nucleic acid sequence.

In some embodiments, the DNA molecule is a circular DNA molecule such as a plasmid, a linear DNA molecule, or a concatemer DNA molecule.

The present invention further relates to a method of *in vitro* transcription of a DNA molecule, comprising the steps of:
(i) contacting at least one DNA molecule, or composition thereof, as described herein, with at least one RNA polymerase, and
(ii) performing *in vitro* transcription of the DNA molecule, thereby providing at least one RNA molecule, in particular at least one messenger RNA (mRNA), for example a self-amplifying messenger RNA, comprising the nucleic acid sequence of interest.

The present invention further relates to a recombinant host cell or microorganism or viral particle comprising the DNA molecule, or composition thereof, as described herein.

### DEFINITIONS

In the present invention, the following terms have the following meanings:
"**About**", when preceding a figure, means plus or less 10% of the value of said figure.

"**And/Or**" refers to and encompasses any and all possible combinations of one or more of the associated listed items, as well as the lack of combinations when interpreted in the alternative ("**or**").

"**At least one**" includes 1, 2, 3, 4, 5, 6, 7, 8, 9, 10, 25, 50, 75, 100, 250, 500, 750, 10³,10⁴, 10⁵, 10⁶, 10⁷, 10⁸, 10⁹, 10¹⁰, 10¹¹, 10¹², 10¹³, 10¹⁴, 10¹⁵ or more.

"**At least two**" includes 2, 3, 4, 5, 6, 7, 8, 9, 10, 25, 50, 75, 100, 250, 500, 750, 10³, 10⁴, 10⁵, 10⁶, 10⁷, 10⁸, 10⁹, 10¹⁰, 10¹¹, 10¹², 10¹³, 10¹⁴, 10¹⁵ or more.

"**Cleavage**", "**cleaving**", "**cleave**", "**cutting**", "**cut**" refer to the breakage of the covalent backbone of a DNA molecule. Cleavage can be initiated by a variety of methods including, but not limited to, enzymatic or chemical hydrolysis of a phosphodiester bond, preferably by a restriction enzyme. Both single-stranded cleavage and double-stranded cleavage are possible, and double-stranded cleavage can occur as a result of two distinct single-stranded cleavage events. DNA cleavage can result in the production of either blunt ends or staggered ends.

"**Comprising**", "**comprises**" and "**comprised of**" are used herein are synonymous with "**including**", "**includes**" or "**containing**", "**contains**", and are inclusive or open-ended and do not exclude additional, non-recited members, elements or method steps. These terms also encompass "**consisting of**"**.**

"**Flanked**" or "**flanking**" means that a first nucleic acid such as a nucleic acid sequence of formula (I), or **SEQ ID NO: 1** is adjacent to a second nucleic acid sequence such as a nucleic acid sequence of interest, a poly(dA) or poly(dT) nucleic acid sequence, a regulatory sequence, or a 5' UTR and 3' UTR. The first nucleic acid sequence may be separated from the second nucleic acid sequence by 1000 nucleotides, 500 nucleotides, 100 nucleotides, 50 nucleotides, 20 nucleotides, 19 nucleotides, 18 nucleotides, 17 nucleotides, 16 nucleotides, 15 nucleotides, 14 nucleotides, 13 nucleotides, 12 nucleotides, 11 nucleotides, 10 nucleotides, 9 nucleotides, 8 nucleotides, 7 nucleotides, 6 nucleotides, 5 nucleotides, 4 nucleotides, 3 nucleotides, 2 nucleotides, 1 nucleotide, or 0 nucleotide.

"**Gene**" or "**transgene**" refer to a DNA region or sequence encoding a peptide, polypeptide, or protein (*i.e.,* a coding region), typically a DNA region or sequence that comprises at least one open reading frame. The term may also include DNA regions which do not *per se* encode a protein (*i.e.,* a non-coding region). The latter include, *e.g*., regions transcribed into functional non-coding RNA molecules (*e.g*., transfer RNA, ribosomal RNA, regulatory RNA, etc.). Other non-coding regions may serve as architectural elements (*e.g*., scaffold/matrix attachment region), as origins of DNA replication, as centromeres or telomeres, etc. Within the scope of the present invention, the gene may be referred to as a gene of interest (GoI) or the transgene may be referred to as a transgene of interest (ToI). The gene, GoI, transgene, or ToI may have a length from 10 nucleic acids to 2,500,000 nucleic acids, from 100 nucleic acids to 10,000 nucleic acids, from 300 nucleic acids to 3,000 nucleic acids, or from 1,000 nucleic acids to 2,000 nucleic acids. The gene, GoI, transgene, or ToI may encode a peptide, polypeptide or protein having a length from 2 amino acids to 40,000 amino acids, from 10 amino acids to 10,000 amino acids, from 50 amino acids to 1,000 amino acids, or from 100 amino acids to 800 amino acids. The peptide, polypeptide or protein may be, illustratively, a soluble or hydrophilic peptide, polypeptide or protein, or may be a membrane, transmembrane or hydrophobic peptide, polypeptide or protein. The peptide, polypeptide or protein may be, illustratively, an antibody, a contractile protein, an enzyme, a hormone, a structural protein, a storage protein, or a transport protein such as a channel, a transporter, or a soluble carrier.

"**Nucleic acid sequence**" and "**nucleotide sequence**" may be used interchangeably to refer to any molecule composed of, or comprising, monomeric nucleotides. A nucleic acid may be an oligonucleotide or a polynucleotide. A nucleotide sequence may be a DNA, RNA, or a mix thereof. A nucleotide sequence may be chemically-modified or artificial. Ribonucleic acid (RNA) molecules encompass in particular messenger RNA (mRNA), typically resulting from the transcription of a DNA sequence; mRNA may be engineered to self-replicate, and such mRNA are termed "self-amplifying RNA" (saRNA), "self-amplifying messenger RNA", "self-replicating RNA" (srRNA), or "self-replicating messenger RNA".

"**Poly(dA)**", "**poly(dA) nucleic acid sequence**" or "**poly(dA) tail**" refers to a nucleic acid sequence consisting of a succession of adenosine, preferably at least 5 adenosines, at least 10 adenosines, at least 50 adenosines, at least 100 adenosines, at least 200 adenosines, or more adenosines, more preferably about 50 adenosines, about 100 adenosines, about 150 adenosines, about 200 adenosines, or about 250 adenosines.

"**Poly(dT)**", "**poly(dT) nucleic acid sequence**" or "**poly(dT) tail**" refers to a nucleic acid sequence consisting of a succession of thymidine, preferably at least 5 thymidine, at least 10 thymidine, at least 50 thymidine, at least 100 thymidine, at least 200 thymidine, or more thymidine, more preferably about 50 thymidine, about 100 thymidine, about 150 thymidine, about 200 thymidine, or about 250 thymidine.

"**Poly(dA/dT)**", "**poly(dA/dT) nucleic acid sequence**" or "**poly(dA/dT) tail**" are considered herein as synonymous and may thus refer to either a poly dA nucleic acid sequence or a poly dT nucleic acid sequence of any given length; in particular of any length that is compatible with *in vivo* cell (e.g. eukaryotic cell) translation.

"**Type II restriction enzymes**" refer herein to the class of enzymes that cleaves DNA at specific recognition sites, typically short palindromic sequences of about 4-8 base pairs; typically, type II restriction enzymes cleave at or near their restriction sites, and usually make double-stranded cuts, producing either blunt ends or sticky (cohesive) ends with overhangs. Type IIB and IIS restriction enzymes cleave a catalytic site which is different from their recognition site - these two sites are physically separated, *i.e*., these enzymes go to the recognition site and then cut/cleave the catalytic site that is located elsewhere, in 5' or 3' of the first site. Typically, type IIP enzymes identify and cut within symmetric (or 'palindromic') DNA sequences that are 4 to 8 base pairs long. Type IIS enzymes typically bind to DNA as single units, recognize asymmetric sequences, and cleave outside of these sequences, usually within one to two turns of the DNA helix. Type IIC enzymes are multifunctional, combining both restriction and modification activities. They contain three domains: one for cleavage, one for methylation, and one for sequence recognition, used by both activities. These enzymes also cut outside their recognition sequences, with a longer "reach" compared to Type IIS enzymes, usually spanning one to two turns of the DNA helix. Most Type IIC enzymes create 2-base 3'-overhangs when they cleave. Type IIB restriction endonucleases typically cleave DNA at both sides of the recognition sequence.

### DETAILED DESCRIPTION

This invention relates to a deoxyribonucleic acid (DNA) molecule, or composition thereof, comprising at least one nucleic acid sequence selected from the group consisting of **SEQ ID NO: 1** to **SEQ ID NO: 57.** In particular, this invention relates to a deoxyribonucleic acid (DNA) molecule, or composition thereof, comprising at least one nucleic acid sequence of formula (I), or **SEQ ID NO 1:**
(I) X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆; wherein:
X₁ is G;
X₂ is selected from a group consisting of: A, C or G;
X₃ is selected from a group consisting of: A, G or T;
X₄ is selected from a group consisting of: A, C, G or T;
X₅ is selected from a group consisting of: A, C or T;
X₆ is selected from a group consisting of: A, C, G or T;
X₇ is selected from a group consisting of: C or G;
X₈ is selected from a group consisting of: A or G;
X₉ is selected from a group consisting of: G or T;
X₁₀ is selected from a group consisting of: A, C, G or T;
X₁₁ is selected from a group consisting of: A, C or T;
X₁₂ is selected from a group consisting of: C, G or T;
X13 is C;
X₁₄ is selected from a group consisting of: A, G or T;
X₁₅ is selected from a group consisting of: A or C;
X₁₆ is C;
X₁₇ is T;
X₁₈ is selected from a group consisting of: C, G or T;
X₁₉ is selected from a group consisting of: A or C;
X₂₀ is selected from a group consisting of: A or G;
X₂₁ is selected from a group consisting of: C, G or T;
X₂₂ is selected from a group consisting of: C or T;
X₂₃ is selected from a group consisting of: C or G;
X24 is G;
X₂₅ is selected from a group consisting of: A or G;
X26 is C;

In some embodiments, the DNA molecule, or composition thereof, according to the invention comprises at least one nucleic acid sequence of formula (I), or **SEQ ID NO 1,** may in particular, be characterized in that it comprises at least 26 nucleotides, at least 30 nucleotides, at least 35 nucleotides, at least 40 nucleotides, at least 45 nucleotides, at least 50 nucleotides, at least 55 nucleotides, at least 60 nucleotides, at least 65 nucleotides, at least 70 nucleotides, at least 75 nucleotides, at least 80 nucleotides, at least 85 nucleotides, at least 90 nucleotides, at least 95 nucleotides, at least 100 nucleotides, at least 150 nucleotides, at least 200 nucleotides, at least 250 nucleotides, at least 300 nucleotides, at least 350 nucleotides, at least 400 nucleotides, at least 450 nucleotides, at least 500 nucleotides, at least 600 nucleotides, at least 700 nucleotides, at least 800 nucleotides, at least 900 nucleotides, at least 1000 nucleotides, at least 5000 nucleotides, at least 10000 nucleotides, or more.

In some embodiments, the DNA molecule or composition thereof comprises at least one nucleic acid sequence of formula (I):
(I) X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆; wherein:
X₁ is G;
X₂ is selected from a group consisting of: A, C or G;
X₃ is selected from a group consisting of: A, G or T;
X₄ is selected from a group consisting of: A, C, G or T;
X₅ is selected from a group consisting of: A, C or T;
X₆ is selected from a group consisting of: A, C, G or T;
X₇ is selected from a group consisting of: C or G;
X₈ is selected from a group consisting of: A or G;
X₉ is selected from a group consisting of: G or T;
X₁₀ is selected from a group consisting of: A, C, G or T;
X₁₁ is selected from a group consisting of: A, C or T;
X₁₂ is selected from a group consisting of: C, G or T;
X13 is C;
X₁₄ is selected from a group consisting of: A, G or T;
X₁₅ is selected from a group consisting of: A or C;
X₁₆ is C;
X₁₇ is T;
X₁₈ is selected from a group consisting of: C, G or T;
X₁₉ is selected from a group consisting of: A or C;
X₂₀ is selected from a group consisting of: A or G;
X₂₁ is selected from a group consisting of: C, G or T;
X₂₂ is selected from a group consisting of: C or T;
X₂₃ is selected from a group consisting of: C or G;
X₂₄ is G;
X₂₅ is selected from a group consisting of: A or G;
X₂₆ is C;
wherein the DNA molecule comprises at least 26 nucleotides, at least 50 nucleotides, at least 100 nucleotides, at least 1000 nucleotides, at least 10000 nucleotides, at least 100000 nucleotides, or at least 1000000 nucleotides.

In some embodiments, the DNA molecule or composition thereof comprises **SEQ ID NO: 1** (GVDNHNSRKNHBCDMCTBMRBYSGRC) (**SEQ ID NO: 1**);
R being A or G; Y being C or T; M being A or C; K being G or T; S being C or G; W being A or T; B being C, G or T; D being A, G or T; H being A, C or T; V being A, C or G; N being A, C, G or T.

In some embodiments, the DNA molecule or composition thereof comprises **SEQ ID NO: 1** (GVDNHNSRKNHBCDMCTBMRBYSGRC) (**SEQ ID NO: 1**);
R being A or G; Y being C or T; M being A or C; K being G or T; S being C or G; W being A or T; B being C, G or T; D being A, G or T; H being A, C or T; V being A, C or G; N being A, C, G or T;
wherein the DNA molecule comprises at least 50 nucleotides; for example wherein the DNA molecule comprises at least 50, 51, 52, 53, 54 or 55 nucleotides, for example at least 60 nucleotides, at least 65 nucleotides, at least 70 nucleotides, at least 75 nucleotides, at least 80 nucleotides, at least 85 nucleotides, at least 90 nucleotides, at least 95 nucleotides, at least 100 nucleotides, at least 150 nucleotides, at least 200 nucleotides, at least 250 nucleotides, at least 300 nucleotides, at least 350 nucleotides, at least 400 nucleotides, at least 450 nucleotides, at least 500 nucleotides, at least 600 nucleotides, at least 700 nucleotides, at least 800 nucleotides, at least 900 nucleotides, at least 1000 nucleotides, at least 5000 nucleotides, at least 10000 nucleotides, or more.

It will be understood that formula (I) or **SEQ ID NO: 1 is a** consensus sequence that encompasses the sequences **SEQ ID NO: 2** to **SEQ ID NO: 57.** Therefore, within the scope of the present disclosure, the expression "formula (I) or **SEQ ID NO: 1**" may refer, in particular, to the nucleic acid sequence of any of **SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, and SEQ ID NO: 57.**

In some embodiments, the DNA molecule or composition thereof comprises at least one nucleic acid sequence selected from the group consisting of **SEQ ID NO: 2** and **SEQ ID NO: 3.** In some embodiments, the DNA molecule or composition thereof comprises at least one nucleic acid sequence selected from the group consisting of **SEQ ID NO: 2** and **SEQ ID NO: 3.**

In some embodiments, the DNA molecule or composition thereof comprises at least one nucleic acid sequence of formula (II) or **SEQ ID NO: 2:**
(II) X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆; wherein:
X₁ is G;
X₂ is selected from a group consisting of: A, C or G;
X₃ is selected from a group consisting of: A, G or T;
X₄ is selected from a group consisting of: A, C, G or T;
X₅ is selected from a group consisting of: A, C or T;
X₆ is selected from a group consisting of: A, C, G or T;
X₇ is selected from a group consisting of: C or G;
X₈ is selected from a group consisting of: A or G;
X₉ is selected from a group consisting of: G or T;
X₁₀ is selected from a group consisting of: A, C, G or T;
X₁₁ is selected from a group consisting of: A, C or T;
X₁₂ is selected from a group consisting of: C, G or T;
X13 is C;
X₁₄ is selected from a group consisting of: G or T;
X₁₅ is selected from a group consisting of: A or C;
X₁₆ is C;
X₁₇ is T;
X₁₈ is selected from a group consisting of: C, G or T;
X₁₉ is selected from a group consisting of: A or C;
X₂₀ is A;
X₂₁ is selected from a group consisting of: C or G;
X₂₂ is T;
X23 is C;
X24 is G;
X₂₅ is selected from a group consisting of: A or G;
X26 is C;
wherein the DNA molecule comprises at least 26 nucleotides, at least 50 nucleotides, at least 100 nucleotides, at least 1000 nucleotides, at least 10000 nucleotides, at least 100000 nucleotides, or at least 1000000 nucleotides.

In some embodiments, the DNA molecule or composition comprises **SEQ ID NO: 2** (GVDNHNSRKNHBCKMCTBMASTCGGC) (SEQ ID NO: 2);
R being A or G; M being A or C; K being G or T; S being C or G; W being A or T; B being C, G or T; D being A, G or T; H being A, C or T; V being A, C or G; N being A, C, G or T;
wherein the DNA molecule comprises at least 26 nucleotides, at least 50 nucleotides, at least 100 nucleotides, at least 1000 nucleotides, at least 10000 nucleotides, at least 100000 nucleotides, or at least 1000000 nucleotides.

In some embodiments, the DNA molecule or composition thereof comprises at least one nucleic acid sequence of formula (III) or **SEQ ID NO: 3:**
(III) X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆; wherein:
X₁ is G;
X₂ is A;
X₃ is selected from a group consisting of: A or G;
X₄ is selected from a group consisting of: A or G;
X₅ is selected from a group consisting of: A or C;
X₆ is selected from a group consisting of: C or G;
X₇ is selected from a group consisting of: C or G;
X₈ is A;
X₉ is selected from a group consisting of: G or T;
X₁₀ is selected from a group consisting of: A or T;
X₁₁ is selected from a group consisting of: C or T;
X₁₂ is selected from a group consisting of: C or G;
X13 is C;
X₁₄ is selected from a group consisting of: A or G;
X₁₅ is C;
X₁₆ is C;
X₁₇ is selected from a group consisting of: A, C, G or T;
X₁₈ is selected from a group consisting of: A, C, G or T;
X₁₉ is selected from a group consisting of: A, C, G or T;
X₂₀ is G;
X₂₁ is T;
X₂₂ is selected from a group consisting of: C or T;
X23 is G;
X24 is G;
X₂₅ is A;
X₂₆ is selected from a group consisting of: A, C, G or T;
wherein the DNA molecule comprises at least 26 nucleotides, at least 50 nucleotides, at least 100 nucleotides, at least 1000 nucleotides, at least 10000 nucleotides, at least 100000 nucleotides, or at least 1000000 nucleotides.

In some embodiments, the DNA molecule or composition thereof comprises **SEQ ID NO: 3** (GARRMSSAKWYSCRCCNNNGTYGGAN) (**SEQ ID NO: 3**); R being A or G; Y being C or T; M being A or C; K being G or T; S being C or G; W being A or T; N being A, C, G or T;
wherein the DNA molecule comprises at least 26 nucleotides, at least 50 nucleotides, at least 100 nucleotides, at least 1000 nucleotides, at least 10000 nucleotides, at least 100000 nucleotides, or at least 1000000 nucleotides.

In some embodiments, the DNA molecule or composition thereof comprises at least one nucleic acid sequence selected from the group consisting of SEQ ID NO: 4 to **SEQ ID NO: 57.** In some embodiments, the DNA molecule or composition thereof comprises at least one nucleic acid sequence selected from the group consisting of **SEQ ID NO: 4** to **SEQ ID NO: 57.**

In some embodiments, the DNA molecule or composition thereof comprises at least one nucleic acid sequence selected from the group consisting of **SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56,** and **SEQ ID NO: 57.**

In some embodiments, the DNA molecule or composition thereof comprises a plurality (i.e. at least two) nucleic acid sequence selected from the group consisting of **SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56,** and **SEQ ID NO: 57.**

In some embodiments, the DNA molecule or composition thereof comprises at least one nucleic acid sequence selected from the group consisting of **SEQ ID NO: 4** to **SEQ ID NO: 57;** wherein the DNA molecule comprises at least 26 nucleotides, at least 50 nucleotides, at least 100 nucleotides, at least 1000 nucleotides, at least 10000 nucleotides, at least 100000 nucleotides, or at least 1000000 nucleotides.

In some embodiments, the DNA molecule or composition thereof comprises at least one nucleic acid sequence selected from the group consisting of **SEQ ID NO: 5** to **SEQ ID NO: 57.**

In some embodiments, the DNA molecule or composition thereof comprises at least one nucleic acid sequence selected from the group consisting of **SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56,** and **SEQ ID NO: 57.**

In some embodiments, the DNA molecule or composition thereof comprises at least two nucleic acid sequences selected from the group consisting of **SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56,** and **SEQ ID NO: 57.**

In one embodiment, the DNA molecule or composition thereof comprises at least one nucleic acid sequence **SEQ ID NO: 2.**

In some embodiments, the DNA molecule or composition thereof comprises at least one nucleic acid sequence selected from the group consisting of **SEQ ID NO: 5, SEQ ID NO: 7 to SEQ ID NO: 10, SEQ ID NO: 12 to SEQ ID NO: 39, SEQ ID NO: 41 to SEQ ID NO: 45, SEQ ID NO: 47, SEQ ID NO: 48, SEQ ID NO: 50, SEQ ID NO: 51, SEQ ID NO: 53, SEQ ID NO: 54, SEQ ID NO: 56,** and **SEQ ID NO: 57.**

In some embodiments, the DNA molecule or composition thereof comprises at least two nucleic acid sequences selected from the group consisting of **SEQ ID NO: 5, SEQ ID NO: 7 to SEQ ID NO: 10, SEQ ID NO: 12 to SEQ ID NO: 39, SEQ ID NO: 41 to SEQ ID NO: 45, SEQ ID NO: 47, SEQ ID NO: 48, SEQ ID NO: 50, SEQ ID NO: 51, SEQ ID NO: 53, SEQ ID NO: 54, SEQ ID NO: 56,** and **SEQ ID NO: 57.**

In some embodiments, the DNA molecule or composition thereof comprises at least one nucleic acid sequence selected from the group consisting of **SEQ ID NO: 5, SEQ ID NO: 7 to SEQ ID NO: 10, SEQ ID NO: 12 to SEQ ID NO: 39, SEQ ID NO: 41 to SEQ ID NO: 45, SEQ ID NO: 47, SEQ ID NO: 48, SEQ ID NO: 50, SEQ ID NO: 51, SEQ ID NO: 53, SEQ ID NO: 54, SEQ ID NO: 56,** and **SEQ ID NO: 57;**
wherein the DNA molecule comprises at least 50, 51, 52, 53, 54 or 55 nucleotides, for example at least 60 nucleotides, at least 65 nucleotides, at least 70 nucleotides, at least 75 nucleotides, at least 80 nucleotides, at least 85 nucleotides, at least 90 nucleotides, at least 95 nucleotides, at least 100 nucleotides, at least 150 nucleotides, at least 200 nucleotides, at least 250 nucleotides, at least 300 nucleotides, at least 350 nucleotides, at least 400 nucleotides, at least 450 nucleotides, at least 500 nucleotides, at least 600 nucleotides, at least 700 nucleotides, at least 800 nucleotides, at least 900 nucleotides, at least 1000 nucleotides, at least 5000 nucleotides, at least 10000 nucleotides, or more.

In some embodiments, the DNA molecule or composition thereof comprises at least one nucleic acid sequence selected from the group consisting of **SEQ ID NO: 5, SEQ ID NO: 7, SEQ ID NO: 8, SEQ ID NO: 9, SEQ ID NO: 10, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 41, SEQ ID NO: 42, SEQ ID NO: 43, SEQ ID NO: 44, SEQ ID NO: 45, SEQ ID NO: 47, SEQ ID NO: 48, SEQ ID NO: 50, SEQ ID NO: 51, SEQ ID NO: 53, SEQ ID NO: 54, SEQ ID NO: 56,** and **SEQ ID NO: 57.**

In some embodiments, the DNA molecule or composition thereof comprises at least one nucleic acid sequence selected from the group consisting of **SEQ ID NO: 5, SEQ ID NO: 7, SEQ ID NO: 8, SEQ ID NO: 9, SEQ ID NO: 10, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 41, SEQ ID NO: 42, SEQ ID NO: 43, SEQ ID NO: 44, SEQ ID NO: 45, SEQ ID NO: 47, SEQ ID NO: 48, SEQ ID NO: 50, SEQ ID NO: 51, SEQ ID NO: 53, SEQ ID NO: 54, SEQ ID NO: 56,** and **SEQ ID NO: 57;** wherein the DNA molecule comprises at least 50, 51, 52, 53, 54 or 55 nucleotides, for example at least 60 nucleotides, at least 65 nucleotides, at least 70 nucleotides, at least 75 nucleotides, at least 80 nucleotides, at least 85 nucleotides, at least 90 nucleotides, at least 95 nucleotides, at least 100 nucleotides, at least 150 nucleotides, at least 200 nucleotides, at least 250 nucleotides, at least 300 nucleotides, at least 350 nucleotides, at least 400 nucleotides, at least 450 nucleotides, at least 500 nucleotides, at least 600 nucleotides, at least 700 nucleotides, at least 800 nucleotides, at least 900 nucleotides, at least 1000 nucleotides, at least 5000 nucleotides, at least 10000 nucleotides, or more.

In one embodiment, the DNA molecule or composition thereof comprises at least one nucleic acid sequence **SEQ ID NO: 3.**

In some embodiments, the DNA molecule or composition comprises at least one nucleic acid sequence selected from the group consisting of: **SEQ ID NO: 6, SEQ ID NO: 11, SEQ ID NO: 40, SEQ ID NO: 46, SEQ ID NO: 49, SEQ ID NO: 52,** and **SEQ ID NO: 55.** In some embodiments, the DNA molecule or composition comprises at least one nucleic acid sequence selected from the group consisting of **SEQ ID NO: 6, SEQ ID NO: 11, SEQ ID NO: 40, SEQ ID NO: 46, SEQ ID NO: 49, SEQ ID NO: 52,** and **SEQ ID NO: 55.**

In some embodiments, the DNA molecule or composition thereof comprises at least one nucleic acid sequence selected from the group consisting of: **SEQ ID NO: 4, SEQ ID NO: 40, SEQ ID NO: 50,** and **SEQ ID NO: 53.**

In some embodiments, the DNA molecule or composition comprises at least one nucleic acid sequence selected from the group consisting of **SEQ ID NO: 4, SEQ ID NO: 40, SEQ ID NO: 50,** and **SEQ ID NO: 53;** wherein the DNA molecule comprises at least 50, 51, 52, 53, 54 or 55 nucleotides, for example at least 60 nucleotides, at least 65 nucleotides, at least 70 nucleotides, at least 75 nucleotides, at least 80 nucleotides, at least 85 nucleotides, at least 90 nucleotides, at least 95 nucleotides, at least 100 nucleotides, at least 150 nucleotides, at least 200 nucleotides, at least 250 nucleotides, at least 300 nucleotides, at least 350 nucleotides, at least 400 nucleotides, at least 450 nucleotides, at least 500 nucleotides, at least 600 nucleotides, at least 700 nucleotides, at least 800 nucleotides, at least 900 nucleotides, at least 1000 nucleotides, at least 5000 nucleotides, at least 10000 nucleotides, or more.

In some embodiments, the DNA molecule or composition thereof comprises at least one nucleic acid sequence selected from the group consisting of **SEQ ID NO: 4, SEQ ID NO: 50,** and **SEQ ID NO: 53.** In some embodiments, the DNA molecule or composition comprises at least one nucleic acid sequence selected from the group consisting of **SEQ ID NO: 4** and **SEQ ID NO: 50.** In some embodiments, the DNA molecule or composition comprises at least one nucleic acid sequence selected from the group consisting of **SEQ ID NO: 4** and **SEQ ID NO: 53.**

In one embodiment, the DNA molecule or composition comprises at least one nucleic acid sequence **SEQ ID NO: 4.** In one embodiment, the DNA molecule or composition comprises at least one nucleic acid sequence **SEQ ID NO: 40.** In one embodiment, the DNA molecule or composition comprises at least one nucleic acid sequence **SEQ ID NO: 50.** In one embodiment, the DNA molecule or composition comprises at least one nucleic acid sequence **SEQ ID NO: 53.**

**Table1: sequences disclosed herein (N represents nucleotide A, C, G or T).**

| **SEQ ID NO:** | **Nucleic acid sequence** |
|---|---|
| **1** | GVDNHNSRKNHBCDMCTBMRBYSGRC |
| **2** | GVDNHNSRKNHBCKMCTBMASTCGGC |
| **3** | GARRMSSAKWYSCRCCNNNGTYGGAN |
| **4** | GAGACCCAGTTCCGCCTGCACTCGGC |
| **5** | GAGACCCAGTTCCGCCTCAAGTINNNN |
| **6** | GAGACCCAGTNCCACNNNNGTTGGAN |
| **7** | NCACTGCNNNTCCGCCTGCACTCGGC |
| **8** | NCACTGCNNCATCGCCTGCACTCGGC |
| **9** | GAAGAGNNNCTCCTCCTGCACTCGGC |
| **10** | GAGACCCAGTNGNTACTTCAGTNNNN |
| **11** | GAGACCCAGTNGCATJNNNNGTCGGAN |
| **12** | GAGACCNNNNTCCGCCTGCACTCGGC |
| **13** | GAGACCCAGTTCCGCCTGCACNNNNN |
| **14** | NCACTGCNNCTCCTCCTGCACTCGGC |
| **15** | GCATTCNNNCTCCTCCTGCACTCGGC |
| **16** | GAAGAGCNNNTCCGCCTGCACTCGGC |
| **17** | GAAGAGGATACGNTACTTCAGTNNNN |
| **18** | GAAGAGCNNCTCCTCCTGCACTCGGC |
| **19** | GAGACGGATACGNTACTCCAGTNNNN |
| **20** | NNNNCCCAGTTCCGCCTGCACTCGGC |
| **21** | GAGACCCAGTNGNTACTCCAGTNNNN |
| **22** | GAAGAGGATACGNTACTCAAGTNNNN |
| **23** | GAGACGGATACNNNNCTGCACTCGGC |
| **24** | NCATTGCNNCTCCTCCTGCACTCGGC |
| **25** | GCATTCNNNCATCGCCTGCACTCGGC |
| **26** | NGTCTTCNNCTCCTCCTGCACTCGGC |
| **27** | GAAGAGNNNNTCCGCCTGCACTCGGC |
| **28** | GAGACGNNNNTCCGCCTGCACTCGGC |
| **29** | NNNGCAGGTGTCCGCCTGCACTCGGC |
| **30** | GAGACCNNNCTCCTCCTGCACTCGGC |
| **31** | GAGACCCAGTNNNNNCTGCACTCGGC |
| **32** | GAGACCCAGTTCCGCCNNNNCTCGGC |
| **33** | GCATTCNNNNTCCGCCTGCACTCGGC |
| **34** | GAGACGNNNCTCCTCCTGCACTCGGC |
| **35** | GAAGAGNNNCATCGCCTGCACTCGGC |
| **36** | GAGACGNNNCATCGCCTGCACTCGGC |
| **37** | GAGACGGATACGNTACTTCAGTNNNN |
| **38** | NCATTGCNNNTCCGCCTGCACTCGGC |
| **39** | NNNGCAGGTCTCCTCCTGCACTCGGC |
| **40** | GAGACGGATACCCACNNNNGTTGGAN |
| **41** | GAGACGNNNCATCGCCTGCACTCGGC |
| **42** | NGTCTTCNNCATCGCCTGCACTCGGC |
| **43** | GAAGAGGATACNNNNCTGCACTCGGC |
| **44** | GAGACCCAGTNGNTACTCAAGTNNNN |
| **45** | GAAGAGGATACGNTACTCCAGTNNNN |
| **46** | GAAGAGGATACCCACNNNNGTTGGAN |
| **47** | NGTCTTCNNNTCCGCCTGCACTCGGC |
| **48** | NCATTGCNNCATCGCCTGCACTCGGC |
| **49** | GAGACCCAGTTCCGCCNNNGTNGGAN |
| **50** | GAGACCCAGTTCCGCCTTCAGTINNNN |
| **51** | NNNGCAGGTCATCGCCTGCACTCGGC |
| **52** | GAAGAGGATACGCANNNNNGTCGGAN |
| **53** | GAAGAGCNNCATCGCCTGCACTCGGC |
| **54** | NNNGCAGGTNTCCGCCTGCACTCGGC |
| **55** | GAGACGGATACGCANNNNNGTCGGAN |
| **56** | GAGACGGATACGNTACTCAAGTNNNN |
| **57** | GAGACCCAGTTCCGCCTCCAGTINNNN |

In some embodiments, the DNA molecule or composition thereof comprises a plurality of restriction enzyme sites.

As used herein, the expression "plurality of restriction enzyme sites" is intended to mean 2, 3, 4, 5, or more, restriction enzyme sites.

In some embodiments, the DNA molecule or composition thereof comprises at least 2 restriction enzyme sites. In some embodiments, the DNA molecule or composition comprises at least 3 restriction enzyme sites. In some embodiments, the DNA molecule or composition thereof comprises at least 4 restriction enzyme sites thereof. In some embodiments, the DNA molecule or composition comprises at least 5 restriction enzyme sites.

In some embodiments, the DNA molecule or composition comprises 2 restriction enzyme sites. In some embodiments, the DNA molecule or composition comprises 3 restriction enzyme sites. In some embodiments, the DNA molecule or composition comprises 4 restriction enzyme sites. In some embodiments, the DNA molecule or composition comprises 5 restriction enzyme sites.

In some embodiments, the DNA molecule or composition comprises a plurality of restriction enzyme sites selected from type II restriction enzyme sites, preferably type IIS, type IIC, type IIB and/or type IIP restriction enzyme sites. In some embodiments, the DNA molecule or composition comprises a plurality of restriction enzyme sites selected from type II restriction enzyme sites, preferably selected from type IIS, type IIC and/or type IIP restriction enzyme sites. In some embodiments, the DNA molecule or composition comprises a plurality of restriction enzyme sites selected from type II restriction enzyme sites, preferably selected from type IIC and/or type IIP restriction enzyme sites. In some embodiments, the plurality of restriction enzyme sites comprises or consists of type II restriction enzyme sites. In some embodiments, the plurality of restriction enzyme sites comprises or consists of type IIS, type IIC and/or type IIP restriction enzyme sites. In some embodiments, the plurality of restriction enzyme sites comprises or consists of type IIS restriction enzyme sites. In some embodiments, the plurality of restriction enzyme sites comprises or consists of type IIC restriction enzyme sites. In some embodiments, the plurality of restriction enzyme sites comprises or consists of type IIP restriction enzyme sites. In some embodiments, the plurality of restriction enzyme sites comprises or consists of type IIB restriction enzyme sites.

In some embodiments, the DNA molecule or composition comprises a plurality of restriction enzyme sites selected from the group consisting of AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PleI, SapI, and SfaNI restriction sites. Hence in some embodiments, DNA molecule or composition comprises a plurality of restriction enzyme sites selected from type II restriction enzyme sites, preferably type IIC, type IIS and/or type IIP restriction enzyme sites, more preferably selected from the group consisting of: AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PleI, SapI, and SfaNI restriction sites.

**Table 2: restriction enzyme sites**

| **Restriction enzyme** | **Restriction site (5' to 3') recognized** |
|---|---|
| AcuI | CTGAAG(16/14) |
| AjuI | (7/12)GAANNNNNNNTTGG(11/6) |
| AlwI | GGATC(4/5) |
| BaeI | (10/15)ACNNNNGTAYC(12/7) |
| BbsI | GAAGAC(2/6) |
| BbvI | GCAGC(8/12) |
| BccI | CCATC(4/5) |
| BceAI | ACGGC(12/14) |
| BcgI | (10/12)CGANNNNNNTGC(12/10) |
| BciVI | GTATCC(6/5) |
| BcoDI | GTCTC(1/5) |
| BfuAI | ACCTGC(4/8) |
| BmrI | ACTGGG(5/4) |
| BpmI | CTGGAG(16/14) |
| BpuEI | CTTGAG(16/14) |
| BsaI | YAC/GTR |
| BsaXI | (9/12)ACNNNNNCTCC(10/7) |
| BseRI | GAGGAG(10/8) |
| BsgI | GTGCAG(16/14) |
| BsmAI | GTCTC(1/5) |
| BsmFI | GGGAC(10/14) |
| BsmI | GAATGC(1/-1) |
| BspCNI | CTCAG(9/7) |
| BspMI | ACCTGC(4/8) |
| BspQI | GCTCTTC(1/4) |
| BsrDI | GCAATG(2/0) |
| BsrI | ACTGG(1/-1) |
| BtgZI | GCGATG(10/14) |
| BtsCI | GGATG(2/0) |
| CspCI | (11/13)CAANNNNNGTGG(12/10) |
| EarI | CTCTTC(1/4) |
| EciI | GGCGGA(11/9) |
| Esp3I | CGTCTC(1/5) |
| FauI | CCCGC(4/6) |
| FokI | GGATG(9/13) |
| HgaI | GACGC(5/10) |
| HphI | GGTGA(8/7) |
| HpyAV | CCTTC(6/5) |
| MboII | GAAGA(8/7) |
| MlyI | GAGTC(5/5) |
| MmeI | TCCRAC(20/18) |
| MnlI | CCTC(7/6) |
| NmeAIII | GCCGAG(21/19) |
| PaqCI | CACCTGC(4/8) |
| PleI | GAGTC(4/5) |
| SapI | GCTCTTC(1/4) |
| SfaNI | GCATC(5/9) |

Number in parenthesis refer to the number of N nucleotides corresponding to the cleavage site corresponding to 5'/3' brand respectively. Such enzymes are commercially available; for example, as sold by New England Biolabs^{®}.

In some embodiments, the DNA molecule or composition thereof comprises a plurality of restriction enzyme sites selected from the group consisting of BpuEI, BcoDI, BsaI, BmrI, EciI, NmeAIII, BsmaI, Esp3I, BciVI, MmeI, AcuI, EarI, SapI, and BtgZI restriction sites.

In some embodiments, the DNA molecule or composition thereof comprises a plurality of restriction enzyme sites selected from the group consisting of BcoDI, BsaI, BmrI, EciI, NmeAIII, BsmaI, Esp3I, BciVI, MmeI, AcuI, EarI, SapI, and BtgZI restriction sites.

In some embodiments, the DNA molecule or composition thereof comprises a plurality of restriction enzyme sites selected from the group consisting of BsaI, BmrI, EciI, and NmeAIII restriction sites.

In some embodiments, the DNA molecule or composition thereof comprises a plurality of restriction enzyme sites selected from the group consisting of BcoDI, BsaI, BmrI, EciI, and NmeAIII restriction sites.

In some embodiments, the DNA molecule or composition thereof comprises a plurality of restriction enzyme sites selected from the group consisting of BsmaI, Esp3I, BciVI, and MmeI restriction sites.

In some embodiments, the DNA molecule or composition thereof comprises a plurality of restriction enzyme sites selected from the group consisting of BsaI, BmrI, EciI, and AcuI restriction sites.

In some embodiments, the DNA molecule or composition thereof comprises a plurality of restriction enzyme sites selected from the group consisting of NmeAIII, EarI, SapI, and BtgZI restriction sites.

In some embodiments, the DNA molecule or composition thereof comprises a plurality of restriction enzyme sites selected from the group consisting of BmrI, BpuEI, BsaI, and EciI restriction sites.

In some embodiments, each restriction enzyme of said plurality of restriction enzyme sites shares at least one cleavage site. In some embodiments, at least 2 restriction enzymes from said plurality of restriction enzyme sites shares at least one cleavage site. In some embodiments, the restriction enzymes corresponding to the plurality of restriction enzyme sites cut or cleave the DNA molecule on the same locus or position. In some embodiments, the restriction enzymes corresponding to the plurality of restriction enzyme sites cut or cleave the DNA molecule at a catalytic site which is different from their recognition site. In some embodiments, the restriction enzymes corresponding to the plurality of restriction enzyme sites may first be localized to the recognition site on the DNA molecule, and then cut/cleave the catalytic site that is located elsewhere on the DNA molecule. In certain embodiments, the restriction enzymes corresponding to the plurality of restriction enzyme sites cut or cleave the DNA molecule at both sides of the recognition sequence.

In some embodiments, the DNA molecule or composition thereof comprises at least one nucleic acid sequence selected from the group consisting of **SEQ ID NO: 2, SEQ ID NO: 3,** and **SEQ ID NO: 4,** wherein said at least one nucleic acid sequence comprises at least 4 restriction enzyme sites. In some embodiments, the DNA molecule or composition comprises at least one nucleic acid sequence selected from the group consisting of **SEQ ID NO: 2,** and **SEQ ID NO: 3,** wherein said at least one nucleic acid sequence comprises at least 4 restriction enzyme sites.

In some embodiments, the DNA molecule or composition thereof comprises at least one nucleic acid sequence **SEQ ID NO: 4,** wherein said at least one nucleic acid sequence comprises at least 5 restriction enzyme sites.

In some embodiments, the DNA molecule or composition thereof comprises at least one nucleic acid sequence selected from the group consisting of **SEQ ID NO: 2, SEQ ID NO: 3,** and **SEQ ID NO: 4,** wherein said at least one nucleic acid sequence comprises a plurality of restriction enzyme sites selected from the group consisting of AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PleI, SapI, and SfaNI restriction sites.

In some embodiments, the DNA molecule or composition comprises at least one nucleic acid sequence selected from the group consisting of **SEQ ID NO: 2, SEQ ID NO: 3,** and **SEQ ID NO: 4,** wherein said at least one nucleic acid sequence comprises a plurality of restriction enzyme sites selected from the group consisting of BpuEI, BcoDI, BsaI, BmrI, EciI, NmeAIII, BsmaI, Esp3I, BciVI, MmeI, AcuI, EarI, SapI, and BtgZI restriction sites.

In some embodiments, the DNA molecule or composition comprises at least one nucleic acid sequence selected from the group consisting of **SEQ ID NO: 2, SEQ ID NO: 3,** and **SEQ ID NO: 4,** wherein said at least one nucleic acid sequence comprises a plurality of restriction enzyme sites selected from the group consisting of BcoDI, BsaI, BmrI, EciI, NmeAIII, BsmaI, Esp3I, BciVI, MmeI, AcuI, EarI, SapI, and BtgZI restriction sites.

In some embodiments, the DNA molecule or composition thereof comprises at least one nucleic acid sequence selected from the group consisting of **SEQ ID NO: 2, SEQ ID NO: 3,** and **SEQ ID NO: 4,** wherein said at least one nucleic acid sequence comprises a plurality of restriction enzyme sites selected from the group consisting of BsaI, BmrI, EciI, and NmeAIII restriction sites.

In some embodiments, the DNA molecule or composition thereof comprises at least one nucleic acid sequence selected from the group consisting of **SEQ ID NO: 2, SEQ ID NO: 3,** and **SEQ ID NO: 4,** wherein said at least one nucleic acid sequence comprises a plurality of restriction enzyme sites selected from the group consisting of BmrI, BpuEI, BsaI, and EciI restriction sites.

In some embodiments, the DNA molecule or composition thereof comprises at least one nucleic acid sequence selected from the group consisting of **SEQ ID NO: 4** to **SEQ ID NO: 57,** wherein said at least one nucleic acid sequence comprises a plurality of restriction enzyme sites selected from the group consisting of AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PleI, SapI, and SfaNI restriction sites.

In some embodiments, the DNA molecule or composition thereof comprises at least one nucleic acid sequence selected from the group consisting of **SEQ ID NO: 4** to **SEQ ID NO: 57,** wherein said at least one nucleic acid sequence comprises a plurality of restriction enzyme sites selected from the group consisting of BpuEI, BcoDI, BsaI, BmrI, EciI, NmeAIII, BsmaI, Esp3I, BciVI, MmeI, AcuI, EarI, SapI, and BtgZI restriction sites.

In some embodiments, the DNA molecule or composition thereof comprises at least one nucleic acid sequence selected from the group consisting of **SEQ ID NO: 4** to **SEQ ID NO: 57,** wherein said at least one nucleic acid sequence comprises a plurality of restriction enzyme sites selected from the group consisting of BcoDI, BsaI, BmrI, EciI, NmeAIII, BsmaI, Esp3I, BciVI, MmeI, AcuI, EarI, SapI, and BtgZI restriction sites.

In some embodiments, the DNA molecule or composition comprises at least one nucleic acid sequence selected from the group consisting of **SEQ ID NO: 4** to **SEQ ID NO: 57;** wherein said at least one nucleic acid sequence comprises a plurality of restriction enzyme sites selected from the group consisting of BsaI, BmrI, EciI, and NmeAIII restriction sites.

In some embodiments, the DNA molecule or composition thereof comprises at least one nucleic acid sequence selected from the group consisting of **SEQ ID NO: 4** to **SEQ ID NO: 57;** wherein said at least one nucleic acid sequence comprises a plurality of restriction enzyme sites selected from the group consisting of BmrI, BpuEI, BsaI, and EciI restriction sites.

In some embodiments, the DNA molecule or composition thereof comprises at least one nucleic acid sequence selected from the group consisting of: **SEQ ID NO: 4, SEQ ID NO: 40, SEQ ID NO: 50,** and **SEQ ID NO: 53,** wherein said at least one nucleic acid sequence comprises a plurality of restriction enzyme sites selected from the group consisting of BpuEI, BcoDI, BsaI, BmrI, EciI, NmeAIII, BsmaI, Esp3I, BciVI, MmeI, AcuI, EarI, SapI, and BtgZI restriction sites.

In some embodiments, the DNA molecule or composition comprises at least one nucleic acid sequence selected from the group consisting of: **SEQ ID NO: 4, SEQ ID NO: 40, SEQ ID NO: 50,** and **SEQ ID NO: 53;** wherein said at least one nucleic acid sequence comprises a plurality of restriction enzyme sites selected from the group consisting of BcoDI, BsaI, BmrI, EciI, NmeAIII, BsmaI, Esp3I, BciVI, MmeI, AcuI, EarI, SapI, and BtgZI restriction sites.

In some embodiments, the DNA molecule or composition thereof comprises at least one nucleic acid sequence selected from the group consisting of: **SEQ ID NO: 4, SEQ ID NO: 50,** and **SEQ ID NO: 53;** wherein said at least one nucleic acid sequence comprises a plurality of restriction enzyme sites selected from the group consisting of BsaI, BmrI, EciI, and NmeAIII restriction sites.

In some embodiments, the DNA molecule or composition thereof comprises at least one nucleic acid sequence **SEQ ID NO: 4,** wherein said at least one nucleic acid sequence comprises a plurality of restriction enzyme sites selected from the group consisting of BcoDI, BsaI, BmrI, EciI, and NmeAIII restriction sites. In some embodiments, the DNA molecule or composition comprises at least one nucleic acid sequence **SEQ ID NO: 4,** wherein said at least one nucleic acid sequence comprises a plurality of restriction enzyme sites selected from the group consisting of BmrI, BpuEI, BsaI, and EciI restriction sites.

In some embodiments, the DNA molecule or composition thereof comprises at least one nucleic acid sequence **SEQ ID NO: 40,** wherein said at least one nucleic acid sequence comprises a plurality of restriction enzyme sites selected from the group consisting of BsmaI, Esp3I, BciVI, and MmeI restriction sites.

In some embodiments, the DNA molecule or composition comprises at least one nucleic acid sequence **SEQ ID NO: 50,** wherein said at least one nucleic acid sequence comprises a plurality of restriction enzyme sites selected from the group consisting of BsaI, BmrI, EciI, and AcuI restriction sites.

In some embodiments, the DNA molecule or composition comprises at least one nucleic acid sequence **SEQ ID NO: 53,** wherein said at least one nucleic acid sequence comprises a plurality of restriction enzyme sites selected from the group consisting of NmeAIII, EarI, SapI, and BtgZI restriction sites.

In some embodiments, the DNA molecule comprises at least 26 nucleotides, at least 30 nucleotides, at least 35 nucleotides, at least 40 nucleotides, at least 45 nucleotides, at least 50 nucleotides, at least 55 nucleotides, at least 60 nucleotides, at least 65 nucleotides, at least 70 nucleotides, at least 75 nucleotides, at least 80 nucleotides, at least 85 nucleotides, at least 90 nucleotides, at least 95 nucleotides, at least 100 nucleotides, at least 150 nucleotides, at least 200 nucleotides, at least 250 nucleotides, at least 300 nucleotides, at least 350 nucleotides, at least 400 nucleotides, at least 450 nucleotides, at least 500 nucleotides, at least 600 nucleotides, at least 700 nucleotides, at least 800 nucleotides, at least 900 nucleotides, at least 1000 nucleotides, at least 5000 nucleotides, at least 10000 nucleotides, at least 100000 nucleotides, at least 1000000 nucleotides, or more. In certain embodiments, the DNA molecule comprises from 26 nucleotides to 1000000 nucleotides, from 30 nucleotides to 50000 nucleotides, from 30 nucleotides to 5000 nucleotides, from 30 nucleotides to 1000 nucleotides, or from 30 nucleotides to 500 nucleotides.

In some embodiments, the DNA molecule comprises more than 5000 nucleotides. In some embodiments, the DNA molecule comprises less than 1000000 nucleotides, for example less than 100000 nucleotides.

In some embodiments, the DNA molecule comprising at least one nucleic acid sequence of formula (I), or **SEQ ID NO 1,** comprises at most 30 nucleotides, preferably comprises at most 26 nucleotides. In some embodiments, the at least one nucleic acid sequence of formula (I), or **SEQ ID NO 1,** comprises about 26 nucleotides. In some embodiments, the at least one nucleic acid sequence of formula (I), or **SEQ ID NO 1,** comprises 26 nucleotides.

According to a preferred embodiment, the DNA molecule comprises more than one nucleic acid sequence of formula (I), or **SEQ ID NO 1,** and comprises more than 52 nucleotides in length.

In some embodiments, the DNA molecule or composition thereof comprises at least two nucleic acid sequences selected from the group consisting of **SEQ ID NO: 1** to **SEQ ID NO: 57.** In some embodiments, the DNA molecule or composition comprises at least two nucleic acid sequences of formula (I) or **SEQ ID NO: 1.** In some embodiments, the DNA molecule or composition thereof comprises two nucleic acid sequences selected from the group consisting of **SEQ ID NO: 1** to **SEQ ID NO: 57.** In some embodiments, the DNA molecule or composition thereof comprises two nucleic acid sequences of formula (I) or **SEQ ID NO: 1.**

In some embodiments, the at least two nucleic acid sequences selected from the group consisting of **SEQ ID NO: 1** to **SEQ ID NO: 57,** or the at least two nucleic acid sequences of formula (I) or **SEQ ID NO: 1,** may be the same (*i.e*., identical) or different (*i.e*., having at least one nucleotide that differs from the other in their sequences). In one embodiment, the at least two nucleic acid sequences selected from the group consisting of **SEQ ID NO: 1** to **SEQ ID NO: 57, or** the at least two nucleic acid sequences of formula (I) or **SEQ ID NO: 1,** are the same. In another embodiment, the at least two nucleic acid sequences selected from the group consisting of **SEQ ID NO: 1** to **SEQ ID NO: 57,** or the at least two nucleic acid sequences of formula (I) or **SEQ ID NO: 1,** are different. In some embodiments, the two nucleic acid sequences selected from the group consisting of **SEQ ID NO: 1** to **SEQ ID NO: 57,** or the two nucleic acid sequences of formula (I) or **SEQ ID NO: 1,** may be the same (*i.e*., identical) or different (*i.e.,* having one nucleotide that differs from the other in their sequences). In one embodiment, the two nucleic acid sequences selected from the group consisting of **SEQ ID NO: 1** to **SEQ ID NO: 57,** or the two nucleic acid sequences of formula (I) or **SEQ ID NO: 1,** are the same. In another embodiment, the two nucleic acid sequences selected from the group consisting of **SEQ ID NO: 1** to **SEQ ID NO: 57,** or the two nucleic acid sequences of formula (I) or **SEQ ID NO: 1,** are different.

In some embodiments, the DNA molecule or composition thereof comprises at least two nucleic acid sequences selected from the group consisting of **SEQ ID NO: 1** to **SEQ ID NO: 57,** which may be the same or different. In some embodiments, the DNA molecule or composition comprises at least two nucleic acid sequences of formula (I) or **SEQ ID NO: 1,** which may be the same or different. In some embodiments, the DNA molecule or composition comprises two nucleic acid sequences selected from the group consisting of **SEQ ID NO: 1** to **SEQ ID NO: 57,** which may be the same or different. In some embodiments, the DNA molecule or composition comprises two nucleic acid sequences of formula (I) or **SEQ ID NO: 1,** which may be the same or different.

In one embodiment, the DNA molecule or composition comprises two identical nucleic acid sequences selected from the group consisting of **SEQ ID NO: 1** to **SEQ ID NO: 57.** In another embodiments, the DNA molecule or composition comprises two distinct nucleic acid sequences selected from the group consisting of **SEQ ID NO: 1** to **SEQ ID NO: 57.**

In some embodiments, the DNA molecule is a double-stranded or single stranded molecule. In one embodiment, the DNA molecule is a double-stranded molecule. In another embodiment, the DNA molecule is a single-stranded molecule.

In the case of a double-stranded molecule, it will be apparent to the person skilled in the art that the second strand comprises at least two sequences complementary to the at least two nucleic acid sequences selected from the group consisting of **SEQ ID NO: 1** to **SEQ ID NO: 57,** or the at least two nucleic acid sequences of formula (I) or **SEQ ID NO: 1** present on the first strand. Thus, the present invention also relates to nucleic acid sequences complementary to any nucleic acid sequence selected from the group consisting of **SEQ ID NO: 1** to **SEQ ID NO: 57.**

In some embodiment, the DNA molecule comprises natural nucleotides or non-natural nucleotides, preferably natural nucleotides. Natural nucleotides include adenine, guanine, cytosine, uracil and thymine. Non-limitative examples of non-natural nucleotides include 2'-Fluoro-dATP, 2'-Fluoro-dCTP, 2'-Fluoro-dGTP, 5- 2'-Amino-dCTP, 2'-Azido-dCTP, 2'-Azido-dATP, 2'-Amino-dATP, 5-Bromo-dCTP, 7-Deaza-dATP, 7-Deaza-dGTP, 5-Propynyl-dCTP, 5-Iodo-dCTP, N6-Methyl-dATP, 5-Methyl-dCTP, O6-Methyl-dGTP, N2-Methyl-dGTP, 8-Oxo-dATP, 8-Oxo-dGTP, 2-Thio-dTTP, 5-Hydroxy-dCTP, 4-Thio-dTTP, 2-Thio-dCTP, 6-Thio-dGTP, 8-Chloro-dATP, 5-AA-dCTP, N4-Methyl-dCTP, 5-Hydroxymethyl-dCTP, 5-Propargylamino-dCTP, 5-Carboxy-dCTP, 5-Formyl-dCTP, and the like.

In some embodiments, the DNA molecule or composition thereof further comprises one or more components selected from the group consisting of: 5' Untranslated Region (5' UTR), 3' Untranslated Region (3' UTR), RNA polymerase promoter region; origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof further comprises a plurality of components (i.e. two or more) selected from the group consisting of: 5' Untranslated Region (5' UTR), 3' Untranslated Region (3' UTR), RNA polymerase promoter region; origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition further comprises one or more of the following: 5' Untranslated Region (5' UTR); 3' Untranslated Region (3' UTR); nucleic acid sequence of interest; a poly(dA) nucleic acid sequence; poly(dT) nucleic acid sequence; at least one regulatory sequence; at least one RNA polymerase promoter region; at least one origin of replication.

In some non-mutually exclusive embodiments, the DNA molecule or composition comprises, or further comprises in addition to the components disclosed above, at least one poly(dA) or poly(dT) nucleic acid sequence.

In some embodiments, the at least one poly(dA) or poly(dT) nucleic acid sequence has a length of at least 5 nucleotides, at least 10 nucleotides, at least 50 nucleotides, at least 100 nucleotides, at least 200 nucleotides, or more. In some embodiments, the DNA molecule or composition comprises a poly(dA) or poly(dT) nucleic acid sequence; wherein the poly(dA) or poly(dT) nucleic acid sequence is characterized by a nucleic acid length of at least 10 nucleotides; in particular at least 20 nucleotides, preferably at least 50 nucleotides. In some embodiments, the DNA molecule or composition comprises a poly(dA/dT) nucleic acid sequence; wherein the poly(dA/dT) nucleic acid sequence is characterized by a nucleic acid length from 10 nucleotides to 150 nucleotides.

In certain embodiments, the poly(dA/dT) nucleic acid sequence has a length from 10 nucleotides to 250 nucleotides, from 10 nucleotides to 200 nucleotides, from 10 nucleotides to 150 nucleotides, from 10 nucleotides to 100 nucleotides, from 10 nucleotides to 50 nucleotides, from 20 nucleotides to 250 nucleotides, from 50 nucleotides to 250 nucleotides, from 100 nucleotides to 250 nucleotides, or from 150 nucleotides to 250 nucleotides. In certain embodiments, the poly(dA/dT) nucleic acid sequence has a length up to 250 nucleotides, up to 200 nucleotides, up to 150 nucleotides, or up to 100 nucleotides.

According to a preferred embodiment a "poly(dA) tail" refers to a nucleic acid sequence consisting of a succession of at least 5 adenosines, at least 10 adenosines, at least 50 adenosines, at least 100 adenosines, at least 200 adenosines, or more adenosines, more preferably about 50 adenosines, about 100 adenosines, about 150 adenosines, about 200 adenosines, or about 250 adenosines. According to a preferred embodiment, a "poly(dT) tail" refers to a nucleic acid sequence consisting of a succession of at least 5 thymidines, at least 10 thymidines, at least 50 thymidines, at least 100 thymidines, at least 200 thymidines, or more thymidines, more preferably about 50 thymidines, about 100 thymidines, about 150 thymidine, about 200 thymidines, or about 250 thymidines.

In some embodiments, the DNA molecule or composition thereof further comprises a 5' Untranslated Region (5' UTR) and/or a 3' Untranslated Region (3' UTR). In some embodiments, the 5' UTR and/or 3' UTR has a length of at least 5 nucleotides, at least 10 nucleotides, at least 50 nucleotides, at least 100 nucleotides, at least 200 nucleotides, or more.

In some embodiments, the DNA molecule or composition thereof further comprises at least one nucleic acid sequence of interest. In some embodiments, the at least one nucleic acid sequence of interest comprises or consists of a gene of interest or fragment thereof, a transgene of interest or fragment thereof, or a regulatory sequence of interest or fragment thereof. In some embodiments, the at least one nucleic acid sequence of interest comprises or consists of at least one gene of interest or fragment thereof. In some embodiments, the nucleic acid sequence of interest comprises or consists of at least one transgene of interest or fragment thereof. It will be apparent to the person skilled in the art that the gene or transgene may be transcribed into a messenger RNA (mRNA), in particular a self-amplifying RNA (saRNA) (interchangeably referred to as self-replicating RNA, srRNA), and may be translated into a peptide, polypeptide or protein.

In some embodiments, the nucleic acid sequence of interest has a length from 50 nucleotides to 5000 nucleotides, from 50 nucleotides to 500 nucleotides, or from 500 nucleotides to 5000 nucleotides.

In certain embodiments, the at least one gene or transgene encodes a peptide or protein useful for treating and/or preventing a disease. In certain embodiments, the at least one gene or transgene encodes a microbial peptide or protein or a fragment thereof, preferably a bacterial, viral or fungal peptide or protein or a fragment thereof. In certain embodiments, the at least one gene or transgene encodes a cancerous or tumoral peptide or protein or a fragment thereof. In certain embodiments, the at least one gene or transgene encodes a peptide or protein involved in a genetic disease, or a fragment thereof.

In some embodiments, the gene is a gene of interest (GoI), and/or the transgene is a transgene of interest (ToI).

In some embodiments, the gene, GoI, transgene, or ToI has a length from 10 nucleic acids to 2,500,000 nucleic acids, from 100 nucleic acids to 10,000 nucleic acids, from 300 nucleic acids to 3,000 nucleic acids, or from 1,000 nucleic acids to 2,000 nucleic acids. In some embodiments, the gene, GoI, transgene, or ToI has a length of at least 10 nucleic acids, at least 100 nuclei acids, at least 1000 nucleic acids, at least 10000 nucleic acids, at least 100000 nucleic acids, or at least 1000000 nucleic acids. In some embodiments, the gene, GoI, transgene, or ToI has a length of at most 10 nucleic acids, at most 100 nuclei acids, at most 1000 nucleic acids, at most 10000 nucleic acids, at most 100000 nucleic acids, or at most 1000000 nucleic acids.

In some embodiments, the gene, GoI, transgene, or ToI encodes a peptide, polypeptide or protein having a length from 2 amino acids to 40,000 amino acids, from 10 amino acids to 10,000 amino acids, from 50 amino acids to 1,000 amino acids, or from 100 amino acids to 800 amino acids. In some embodiments, the gene, GoI, transgene, or ToI encodes a peptide, polypeptide or protein having a length of at least 2 amino acids, at least 10 amino acids, at least 100 amino acids, at least 1000 amino acids, or at least 10000 amino acids. In some embodiments, the gene, GoI, transgene, or ToI encodes a peptide, polypeptide or protein having a length of at most 2 amino acids, at most 10 amino acids, at most 100 amino acids, at most 1000 amino acids, or at most 10000 amino acids.

In some embodiments, the peptide, polypeptide or protein is a soluble or hydrophilic peptide, polypeptide or protein; or a membrane, transmembrane or hydrophobic peptide, polypeptide or protein.

In certain embodiments, the peptide, polypeptide or protein is selected from the group consisting of an antibody, a contractile protein, an enzyme, a hormone, a structural protein, a storage protein, or a transport protein such as a channel, a transporter, or a soluble carrier.

In some embodiments, the gene or transgene is an animal gene or transgene, a mammalian gene or transgene, or a human gene or transgene. In some embodiments, the gene or transgene is a genetically engineered gene or transgene. In some embodiments, the gene or transgene is a codon-optimized transgene.

In some embodiments, the DNA molecule or composition thereof further comprises at least one 5' UTR, at least one 3' UTR, and at least one nucleic acid sequence of interest. In some embodiments, the DNA molecule or composition further comprises at least one poly(dA) or poly(dT) nucleic acid sequence, and at least one nucleic acid sequence of interest.

In some embodiments, the DNA molecule or composition thereof further comprises one or more component selected from the group consisting of origin of replication, antibiotic resistance genes (e.g., AmpR, KanR, TetR, or CmR), multiple cloning sites, promoters (e.g., T7 promoter, CMV promoter, lac promoter), selectable markers, reporter genes, fusion tags, termination sequences, origins of transfer sequences, and regulatory elements.

In some embodiments, the DNA molecule or composition further comprises at least one regulatory sequence. In some embodiments, the at least one regulatory sequence is selected from the group consisting of a promoter, an enhancer, a silencer, an insulator, or a repressor. In some embodiments, the at least one regulatory sequence is a promoter. In some embodiments, the at least one regulatory sequence is a promoter for a DNA polymerase or a RNA polymerase. In some embodiments, the at least one regulatory sequence is a promoter for a RNA polymerase.

In some embodiments, the DNA molecule or composition thereof further comprises at least one RNA polymerase promoter region.

In some embodiments, the DNA molecule or composition thereof further comprises at least one origin of replication.

In some embodiments, the DNA molecule or composition thereof further comprises at least one antibiotic resistance genes, such as AmpR, KanR, TetR, or CmR.

In some embodiments, the DNA molecule or composition thereof further comprises at least one multiple cloning site.

In some embodiments, the DNA molecule or composition thereof further comprises at least one promoter, such as T7 promoter, CMV promoter, lac promoter.

In some embodiments, the DNA molecule or composition thereof further comprises at least one reporter gene.

In some embodiments, the DNA molecule or composition thereof further comprises at least one termination sequence.

In some embodiments, the DNA molecule or composition thereof further comprises at least one origins of transfer sequence.

In some embodiments, the DNA molecule or composition thereof further comprises:
- at least one 5' Untranslated Region (5' UTR) and at least one 3' Untranslated Region (3' UTR);
- at least one nucleic acid sequence of interest; and
- a poly(dA) or poly(dT) nucleic acid sequence.

In some embodiments, the DNA molecule or composition further comprises:
- at least one 5' Untranslated Region (5' UTR) and at least one 3' Untranslated Region (3' UTR);
- at least one regulatory sequence;
- at least one nucleic acid sequence of interest; and
- a poly(dA) or poly(dT) nucleic acid sequence.

In some embodiments, the DNA molecule or composition further comprises:
- at least one 5' Untranslated Region (5' UTR) and at least one 3' Untranslated Region (3' UTR);
- at least one regulatory sequence;
- at least one nucleic acid sequence of interest;
- at least one RNA polymerase promoter region; and
- a poly(dA) or poly(dT) nucleic acid sequence.

In some embodiments, the DNA molecule or composition further comprises:
- at least one 5' Untranslated Region (5' UTR) and at least one 3' Untranslated Region (3' UTR);
- at least one regulatory sequence;
- at least one nucleic acid sequence of interest;
- at least one origin of replication; and
- a poly(dA) or poly(dT) nucleic acid sequence.

In some embodiments, the at least one nucleic acid sequence of interest is flanked by at least one nucleic acid sequence of formula (I), or **SEQ ID NO: 1** as described herein. In some embodiments, the at least one nucleic acid sequence of interest, and the at least one poly(dA) or poly(dT) nucleic acid sequence, are flanked by at least one nucleic acid sequence of formula (I), or **SEQ ID NO: 1.** In some embodiments, the at least one nucleic acid sequence of interest, the at least one poly(dA) or poly(dT) nucleic acid sequence, the at least one regulatory sequence, and the at least one 5' UTR and 3' UTR, are flanked by at least one nucleic acid sequence of formula (I), or **SEQ ID NO: 1** as described herein.

In certain embodiments, the at least one nucleic acid sequence of formula (I), or **SEQ ID NO: 1** as described herein is separated from the at least one nucleic acid sequence of interest, the at least one poly(dA) or poly(dT) nucleic acid sequence, the at least one regulatory sequence, or the at least one 5' UTR and 3' UTR, by 10000 nucleotides, 1000 nucleotides, 500 nucleotides, 100 nucleotides, 50 nucleotides, 40 nucleotides, 30 nucleotides, 20 nucleotides, 19 nucleotides, 18 nucleotides, 17 nucleotides, 16 nucleotides, 15 nucleotides, 14 nucleotides, 13 nucleotides, 12 nucleotides, 11 nucleotides, 10 nucleotides, 9 nucleotides, 8 nucleotides, 7 nucleotides, 6 nucleotides, 5 nucleotides, 4 nucleotides, 3 nucleotides, 2 nucleotides, 1 nucleotide, or 0 nucleotide, preferably 0 nucleotide.

In some embodiments, at least one nucleic acid sequence of formula (I), or **SEQ ID NO: 1,** is localized in 5' of the at least one nucleic acid sequence of interest, and at least one nucleic acid sequence of formula (I), or **SEQ ID NO: 1,** is localized in 3' of the poly(dA/dT) nucleic acid sequence

In some embodiments, the DNA molecule or composition thereof comprises from 5' to 3': a nucleic acid sequence of interest; a poly(dA) or poly(dT) nucleic acid sequence; and a nucleic acid sequence of formula (I), or **SEQ ID NO: 1.**

In some embodiments, the DNA molecule or composition thereof comprises from 5' to 3': a 5'UTR; a nucleic acid sequence of interest; a 3'UTR; a poly(dA) or poly(dT) nucleic acid sequence; and a nucleic acid sequence of formula (I), or **SEQ ID NO: 1.**

In some embodiments, the DNA molecule or composition thereof comprises from 5' to 3': optionally at least one first regulatory sequence; a 5'UTR; a nucleic acid sequence of interest; a 3'UTR; a poly(dA) or poly(dT) nucleic acid sequence; optionally at least one regulatory sequence; and a nucleic acid sequence of formula (I), or **SEQ ID NO: 1.**

In some embodiments, the DNA molecule or composition thereof comprises from 5' to 3': optionally a RNA polymerase promoter region; optionally at least one first regulatory sequence; a 5'UTR; a nucleic acid sequence of interest; a 3'UTR; optionally at least one regulatory sequence; a poly(dA) or poly(dT) nucleic acid sequence; and a nucleic acid sequence of formula (I), or **SEQ ID NO: 1.**

In some embodiments, the DNA molecule or composition thereof comprises from 5' to 3': optionally an origin of replication; optionally at least one first regulatory sequence; a 5'UTR; a nucleic acid sequence of interest; a 3'UTR; optionally at least one regulatory sequence; a poly(dA) or poly(dT) nucleic acid sequence; and a nucleic acid sequence of formula (I), or **SEQ ID NO: 1.**

In some embodiments, the DNA molecule or composition thereof comprises from 5' to 3': a first nucleic acid sequence of formula (I), or **SEQ ID NO: 1;** a nucleic acid sequence of interest; a poly(dA) or poly(dT) nucleic acid sequence; and a second nucleic acid sequence of formula (I), or **SEQ ID NO: 1.**

In some embodiments, the DNA molecule or composition thereof comprises from 5' to 3': a first nucleic acid sequence of formula (I), or **SEQ ID NO: 1;** a 5'UTR; a nucleic acid sequence of interest; a 3'UTR; a poly(dA) or poly(dT) nucleic acid sequence; and a second nucleic acid sequence of formula (I), or **SEQ ID NO: 1,** wherein said first nucleic acid sequence of formula (I), or **SEQ ID NO 1,** and said second nucleic acid sequence of formula (I), or **SEQ ID NO 1,** may be the same or different.

In some embodiments, the DNA molecule or composition comprises from 5' to 3': a first nucleic acid sequence of formula (I), or **SEQ ID NO: 1;** optionally at least one first regulatory sequence; a 5'UTR; a nucleic acid sequence of interest; a 3'UTR; a poly(dA) or poly(dT) nucleic acid sequence; optionally at least one second regulatory sequence; and a second nucleic acid sequence of formula (I), or **SEQ ID NO: 1,** wherein said first nucleic acid sequence of formula (I), or **SEQ ID NO 1,** and said second nucleic acid sequence of formula (I), or **SEQ ID NO 1,** may be the same or different.

In some embodiments, the DNA molecule or composition thereof comprises from 5' to 3': a first nucleic acid sequence of formula (I), or **SEQ ID NO: 1;** optionally a RNA polymerase promoter region; optionally at least one first regulatory sequence; a 5'UTR; a nucleic acid sequence of interest; a 3'UTR; optionally at least one second regulatory sequence; a poly(dA) or poly(dT) nucleic acid sequence; and a second nucleic acid sequence of formula (I), or **SEQ ID NO: 1,** wherein said first nucleic acid sequence of formula (I), or **SEQ ID NO 1,** and said second nucleic acid sequence of formula (I), or **SEQ ID NO 1,** may be the same or different.

In some embodiments, the DNA molecule or composition thereof comprises from 5' to 3': a first nucleic acid sequence of formula (I), or **SEQ ID NO: 1;** optionally an origin of replication; optionally at least one first regulatory sequence; a 5'UTR; a nucleic acid sequence of interest; a 3'UTR; optionally at least one second regulatory sequence; a poly(dA) or poly(dT) nucleic acid sequence; and a second nucleic acid sequence of formula (I), or **SEQ ID NO: 1,** wherein said first nucleic acid sequence of formula (I), or **SEQ ID NO 1,** and said second nucleic acid sequence of formula (I), or **SEQ ID NO 1,** may be the same or different.

In some embodiments, the DNA molecule is a circular DNA molecule such as a plasmid, a linear DNA molecule, or a concatemer DNA molecule. In some embodiments, the DNA molecule is a circular DNA molecule such as a plasmid, or a linear DNA molecule. In some embodiments, the DNA molecule is a linear DNA molecule or a concatemer DNA molecule.

In some embodiments, the DNA molecule is selected from the group consisting of plasmid DNA (pDNA), cosmid DNA, fosmid DNA, bacterial artificial chromosome, human artificial chromosome, DNA-comprising ribonucleoprotein, minicircle DNA, linear DNA, concatemer DNA, and linear covalently closed DNA (LCC DNA). In some embodiments, the DNA molecule is selected from the group consisting of plasmid DNA, cosmid DNA, fosmid DNA, bacterial artificial chromosome, human artificial chromosome, concatemer DNA and linear covalently closed DNA.

In some embodiments, the DNA molecule or composition thereof comprises or consists of a nucleic acid sequence as defined hereinafter:
(5') [**SEQ ID NO: 1**]ₓ - GoI - Poly(dA/dT) - [**SEQ ID NO: 1**]_{y} (3')
wherein x is an integer number being 0, 1, 2, 3, 4, or 5, preferably 0 or 1; wherein y is a positive integer number being 1, 2, 3, 4, or 5, preferably 1.

In some embodiments, the DNA molecule or composition thereof comprises or consists of a nucleic acid sequence as defined hereinafter:
(5') [**SEQ ID NO: 1**]ₓ - 5' UTR - GoI - Poly(dA/dT) - [**SEQ ID NO: 1**]_{y} (3')
wherein x is an integer number being 0, 1, 2, 3, 4, or 5, preferably 0 or 1; wherein y is a positive integer number being 1, 2, 3, 4, or 5, preferably 1.

In some embodiments, the DNA molecule or composition thereof comprises or consists of a nucleic acid sequence as defined hereinafter:
(5') [**SEQ ID NO: 1**]ₓ - 5' UTR - GoI - Poly(dA/dT) - 3' UTR - [**SEQ ID NO: 1**]_{y} (3')
wherein x is an integer number being 0, 1, 2, 3, 4, or 5, preferably 0 or 1; wherein y is a positive integer number being 1, 2, 3, 4, or 5, preferably 1.

In some embodiments, the DNA molecule or composition thereof is selected from the group consisting of plasmid DNA, cosmid DNA, fosmid DNA, bacterial artificial chromosome, human artificial chromosome, and linear covalently closed DNA comprising a nucleic acid sequence as defined hereafter:
5' [**SEQ ID NO: 1**]x -5' UTR - GoI - 3' UTR - Poly(dA/dT) - [**SEQ ID NO: 1**]y 3'
wherein x is an integer number being 0, 1, 2, 3, 4, or 5, preferably 0 or 1; wherein y is a positive integer number being 1, 2, 3, 4, or 5, preferably 1, and
wherein the DNA molecule further comprises one or more component selected from the group consisting of origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof is a circular DNA molecule, a linear DNA molecule, or a DNA concatemer molecule, comprising a nucleic acid sequence as defined hereafter:
(5') [**SEQ ID NO: 1**]ₓ - 5' UTR - GoI- 3' UTR - Poly(dA/dT) - [**SEQ ID NO: 1**]_{y} (3')
wherein x is an integer number being 0, 1, 2, 3, 4, or 5, preferably 0 or 1; wherein y is a positive integer number being 1, 2, 3, 4, or 5, preferably 1, and
wherein the DNA molecule further comprises one or more component selected from the group consisting of origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof comprises or consists of a nucleic acid sequence as defined hereinafter:
(5') [first formula (I) sequence]ₓ - GoI - Poly(dA/dT) - [second formula (I) sequence]_{y} (3')
wherein x is an integer number being 0, 1, 2, 3, 4, or 5, preferably 0 or 1; wherein y is a positive integer number being 1, 2, 3, 4, or 5, preferably 1,
wherein the first formula (I) sequence and the second formula (I) sequence may be the same or different.

In some embodiments, the DNA molecule or composition thereof comprises or consists of a nucleic acid sequence as defined hereinafter:
(5') [first formula (I) sequence]ₓ - 5' UTR - GoI - Poly(dA/dT) - [second formula (I) sequence]_{y} (3')
wherein x is an integer number being 0, 1, 2, 3, 4, or 5, preferably 0 or 1; wherein y is a positive integer number being 1, 2, 3, 4, or 5, preferably 1,
wherein the first formula (I) sequence and the second formula (I) sequence may be the same or different.

In some embodiments, the DNA molecule or composition thereof comprises or consists of a nucleic acid sequence as defined hereinafter:
(5') [first formula (I) sequence]ₓ - 5' UTR - GoI - Poly(dA/dT) - 3' UTR - [second formula (I) sequence]_{y} (3')
wherein x is an integer number being 0, 1, 2, 3, 4, or 5, preferably 0 or 1; wherein y is a positive integer number being 1, 2, 3, 4, or 5, preferably 1,
wherein the first formula (I) sequence and the second formula (I) sequence may be the same or different.

In some embodiments, the DNA molecule or composition thereof is selected from the group consisting of plasmid DNA, cosmid DNA, fosmid DNA, bacterial artificial chromosome, human artificial chromosome, and linear covalently closed DNA comprising a nucleic acid sequence as defined hereafter:
5' [first formula (I) sequence]x -5' UTR - GoI - 3' UTR - Poly(dA/dT) - [second formula (I) sequence]y 3'
wherein x is an integer number being 0, 1, 2, 3, 4, or 5, preferably 0 or 1; wherein y is a positive integer number being 1, 2, 3, 4, or 5, preferably 1,
wherein the first formula (I) sequence and the second formula (I) sequence may be the same or different, and
wherein the DNA molecule further comprises one or more component selected from the group consisting of origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof is a circular DNA molecule, a linear DNA molecule, or a DNA concatemer molecule, comprising a nucleic acid sequence as defined hereafter:
(5') [first formula (I) sequence]ₓ - 5' UTR - GoI- 3' UTR - Poly(dA/dT) - [second formula (I) sequence]_{y} (3')
wherein x is an integer number being 0, 1, 2, 3, 4, or 5, preferably 0 or 1; wherein y is a positive integer number being 1, 2, 3, 4, or 5, preferably 1,
wherein the first formula (I) sequence and the second formula (I) sequence may be the same or different, and
wherein the DNA molecule further comprises one or more component selected from the group consisting of origin of replication, antibiotic resistance genes, multiple cloning sites, promoters, selectable markers, reporter genes, fusion tags, termination sequences, origins of transfer sequences, and regulatory elements.

In one embodiment, the DNA molecule is a circular DNA molecule, preferably a plasmid. In some embodiments, the DNA molecule is selected from the group consisting of plasmid DNA, cosmid DNA, and fosmid DNA. In some embodiments, the DNA molecule is a plasmid.

In another embodiment, the DNA molecule is a linear DNA. In another embodiment, the DNA molecule is a concatemer DNA molecule.

In certain embodiments, the DNA molecule is a double-stranded circular DNA molecule, preferably a plasmid, comprising from 5' to 3': a first nucleic acid sequence of formula (I), or **SEQ ID NO: 1;** optionally at least one first regulatory sequence; a 5'UTR; a nucleic acid sequence of interest; a 3'UTR; a poly(dA) or poly(dT) nucleic acid sequence; optionally at least one second regulatory sequence; and a second nucleic acid sequence of formula (I), or **SEQ ID NO: 1,** wherein said first nucleic acid sequence of formula (I), or **SEQ ID NO 1,** and said second nucleic acid sequence of formula (I), or **SEQ ID NO 1,** may be the same or different.

In certain embodiments, the DNA molecule is a concatemer DNA molecule.

In some embodiments, the composition comprising the DNA molecule or composition thereof as described herein further comprises one or more of an aqueous buffer, ions, or a preservative agent.

In some embodiments, the restriction enzymes corresponding to the plurality of restriction enzyme sites cut or cleave the DNA molecule on the same locus or position. In some embodiments, the plurality of restrictions enzymes as described herein, have at least one common cleavage site, or the same cleavage site. In some embodiments, the plurality of restrictions enzymes may recognize distinct restriction sites, and have the same cleavage site. In some embodiments, the restriction enzymes corresponding to the plurality of restriction enzyme sites cut or cleave the DNA molecule at a catalytic site which is different from their recognition site. In some embodiments, the restriction enzymes corresponding to the plurality of restriction enzyme sites may first be localized to the recognition site on the DNA molecule, and then cut/cleave the catalytic site that is located elsewhere on the DNA molecule. In certain embodiments, the restriction enzymes corresponding to the plurality of restriction enzyme sites cut or cleave the DNA molecule at both sides of the recognition sequence.

In some embodiment, the DNA molecule or composition as described herein is not a naturally-occurring DNA molecule. In some embodiment, the DNA molecule or composition as described herein is not a DNA molecule found in nature.

In some embodiments, the DNA molecule or composition thereof comprises at least one nucleic acid sequence of formula (I) or **SEQ ID NO: 1:**
(I) X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆; wherein:
X₁ is G;
X₂ is selected from a group consisting of: A, C or G;
X₃ is selected from a group consisting of: A, G or T;
X₄ is selected from a group consisting of: A, C, G or T;
X₅ is selected from a group consisting of: A, C or T;
X₆ is selected from a group consisting of: A, C, G or T;
X₇ is selected from a group consisting of: C or G;
X₈ is selected from a group consisting of: A or G;
X₉ is selected from a group consisting of: G or T;
X₁₀ is selected from a group consisting of: A, C, G or T;
X₁₁ is selected from a group consisting of: A, C or T;
X₁₂ is selected from a group consisting of: C, G or T;
X13 is C;
X₁₄ is selected from a group consisting of: A, G or T;
X₁₅ is selected from a group consisting of: A or C;
X₁₆ is C;
X₁₇ is T;
X₁₈ is selected from a group consisting of: C, G or T;
X₁₉ is selected from a group consisting of: A or C;
X₂₀ is selected from a group consisting of: A or G;
X₂₁ is selected from a group consisting of: C, G or T;
X₂₂ is selected from a group consisting of: C or T;
X₂₃ is selected from a group consisting of: C or G;
X24 is G;
X₂₅ is selected from a group consisting of: A or G;
X₂₆ is C;

wherein the DNA molecule or composition thereof comprises at least 50 nucleotides, at least 100 nucleotides, at least 1000 nucleotides, at least 10000 nucleotides, at least 100000 nucleotides, or at least 1000000 nucleotides;
wherein the DNA molecule or composition thereof comprises or consists of a nucleic acid sequence as defined hereinafter:
   (5') [first sequence of formula (I) or **SEQ ID NO: 1**]ₓ - GoI - Poly(dA/dT) - [second sequence of formula (I) or **SEQ ID NO: 1**]_{y} (3')
wherein x is an integer number being 0, 1, 2, 3, 4, or 5, preferably 0 or 1; wherein y is a positive integer number being 1, 2, 3, 4, or 5, preferably 1,
wherein the first sequence of formula (I) or **SEQ ID NO: 1** and the second sequence of formula (I) or **SEQ ID NO: 1** may be the same or different, and
optionally wherein the DNA molecule or composition thereof further comprises one or more component selected from the group consisting of 5' Untranslated Region (5' UTR), 3' Untranslated Region (3' UTR), RNA polymerase promoter region, origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof comprises at least one nucleic acid sequence of formula (II) or **SEQ ID NO: 2:**
(II) X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆; wherein:
X₁ is G;
X₂ is selected from a group consisting of: A, C or G;
X₃ is selected from a group consisting of: A, G or T;
X₄ is selected from a group consisting of: A, C, G or T;
X₅ is selected from a group consisting of: A, C or T;
X₆ is selected from a group consisting of: A, C, G or T;
X₇ is selected from a group consisting of: C or G;
X₈ is selected from a group consisting of: A or G;
X₉ is selected from a group consisting of: G or T;
X₁₀ is selected from a group consisting of: A, C, G or T;
X₁₁ is selected from a group consisting of: A, C or T;
X₁₂ is selected from a group consisting of: C, G or T;
X13 is C;
X₁₄ is selected from a group consisting of: G or T;
X₁₅ is selected from a group consisting of: A or C;
X₁₆ is C;
X₁₇ is T;
X₁₈ is selected from a group consisting of: C, G or T;
X₁₉ is selected from a group consisting of: A or C;
X₂₀ is A;
X₂₁ is selected from a group consisting of: C or G;
X₂₂ is T;
X23 is C;
X24 is G;
X₂₅ is selected from a group consisting of: A or G;
X26 is C;

wherein the DNA molecule or composition thereof comprises at least 50 nucleotides, at least 100 nucleotides, at least 1000 nucleotides, at least 10000 nucleotides, at least 100000 nucleotides, or at least 1000000 nucleotides;
wherein the DNA molecule or composition thereof comprises or consists of a nucleic acid sequence as defined hereinafter:
   (5') [first sequence of formula (II) or **SEQ ID NO: 2**]ₓ - GoI - Poly(dA/dT) - [second sequence of formula (II) or **SEQ ID NO: 2**]_{y} (3')
wherein x is an integer number being 0, 1, 2, 3, 4, or 5, preferably 0 or 1; wherein y is a positive integer number being 1, 2, 3, 4, or 5, preferably 1,
wherein the first sequence of formula (II) or **SEQ ID NO: 2** and the second sequence of formula (II) or **SEQ ID NO: 2** may be the same or different,
preferably wherein the DNA molecule or composition thereof is a circular DNA molecule, a linear DNA molecule, or a concatemer DNA molecule, and
optionally wherein the DNA molecule or composition thereof further comprises one or more component selected from the group consisting of 5' Untranslated Region (5' UTR), 3' Untranslated Region (3' UTR), RNA polymerase promoter region, origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof comprises at least one nucleic acid sequence of formula (III) or **SEQ ID NO: 3:**
(III) X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆; wherein:
X₁ is G;
X₂ is A;
X₃ is selected from a group consisting of: A or G;
X₄ is selected from a group consisting of: A or G;
X₅ is selected from a group consisting of: A or C;
X₆ is selected from a group consisting of: C or G;
X₇ is selected from a group consisting of: C or G;
X₈ is A;
X₉ is selected from a group consisting of: G or T;
X₁₀ is selected from a group consisting of: A or T;
X₁₁ is selected from a group consisting of: C or T;
X₁₂ is selected from a group consisting of: C or G;
X13 is C;
X₁₄ is selected from a group consisting of: A or G;
X₁₅ is C;
X₁₆ is C;
X₁₇ is selected from a group consisting of: A, C, G or T;
X₁₈ is selected from a group consisting of: A, C, G or T;
X₁₉ is selected from a group consisting of: A, C, G or T;
X₂₀ is G;
X₂₁ is T;
X₂₂ is selected from a group consisting of: C or T;
X23 is G;
X24 is G;
X₂₅ is A;
X₂₆ is selected from a group consisting of: A, C, G or T;

wherein the DNA molecule or composition thereof comprises at least 50 nucleotides, at least 100 nucleotides, at least 1000 nucleotides, at least 10000 nucleotides, at least 100000 nucleotides, or at least 1000000 nucleotides;
wherein the DNA molecule or composition thereof comprises or consists of a nucleic acid sequence as defined hereinafter:
   (5') [first sequence of formula (III) or **SEQ ID NO: 3**]ₓ - GoI - Poly(dA/dT) - [second sequence of formula (III) or **SEQ ID NO: 3**]_{y} (3')
wherein x is an integer number being 0, 1, 2, 3, 4, or 5, preferably 0 or 1; wherein y is a positive integer number being 1, 2, 3, 4, or 5, preferably 1,
wherein the first sequence of formula (III) or **SEQ ID NO: 3** and the second sequence of formula (III) or **SEQ ID NO: 3** may be the same or different,
preferably wherein the DNA molecule or composition thereof is a circular DNA molecule, a linear DNA molecule, or a concatemer DNA molecule, and
optionally wherein the DNA molecule or composition thereof further comprises one or more component selected from the group consisting of 5' Untranslated Region (5' UTR), 3' Untranslated Region (3' UTR), RNA polymerase promoter region, origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof comprises at least one nucleic acid sequence selected from the group consisting of **SEQ ID NO: 4** to **SEQ ID NO: 57;**
wherein the DNA molecule or composition thereof comprises at least 50 nucleotides, at least 100 nucleotides, at least 1000 nucleotides, at least 10000 nucleotides, at least 100000 nucleotides, or at least 1000000 nucleotides;
wherein the DNA molecule or composition thereof comprises or consists of a nucleic acid sequence as defined hereinafter:
   (5') [first sequence selected from the group consisting of **SEQ ID NO: 4** to **SEQ ID NO: 57**]ₓ - GoI - Poly(dA/dT) - [second sequence selected from the group consisting of **SEQ ID NO: 4** to **SEQ ID NO: 57**]_{y} (3')
wherein x is an integer number being 0, 1, 2, 3, 4, or 5, preferably 0 or 1; wherein y is a positive integer number being 1, 2, 3, 4, or 5, preferably 1,
wherein the first sequence selected from the group consisting of **SEQ ID NO: 4** to **SEQ ID NO: 57** and the second sequence selected from the group consisting of **SEQ ID NO: 4** to **SEQ ID NO: 57,** may be the same or different,
preferably wherein the DNA molecule or composition thereof is a circular DNA molecule, a linear DNA molecule, or a concatemer DNA molecule, and
optionally wherein the DNA molecule or composition thereof further comprises one or more component selected from the group consisting of 5' Untranslated Region (5' UTR), 3' Untranslated Region (3' UTR), RNA polymerase promoter region, origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof comprises at least one nucleic acid sequence of **SEQ ID NO: 4;**
wherein the DNA molecule or composition thereof comprises at least 50 nucleotides, at least 100 nucleotides, at least 1000 nucleotides, at least 10000 nucleotides, at least 100000 nucleotides, or at least 1000000 nucleotides;
wherein the DNA molecule or composition thereof comprises or consists of a nucleic acid sequence as defined hereinafter:
   (5') [**SEQ ID NO: 4**]ₓ - GoI - Poly(dA/dT) - [**SEQ ID NO: 4**]_{y} (3')
wherein x is an integer number being 0, 1, 2, 3, 4, or 5, preferably 0 or 1; wherein y is a positive integer number being 1, 2, 3, 4, or 5, preferably 1,
preferably wherein the DNA molecule or composition thereof is a circular DNA molecule, a linear DNA molecule, or a concatemer DNA molecule, and
optionally wherein the DNA molecule or composition thereof further comprises one or more component selected from the group consisting of 5' Untranslated Region (5' UTR), 3' Untranslated Region (3' UTR), RNA polymerase promoter region, origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof comprises at least one nucleic acid sequence of **SEQ ID NO: 40;**
wherein the DNA molecule or composition thereof comprises at least 50 nucleotides, at least 100 nucleotides, at least 1000 nucleotides, at least 10000 nucleotides, at least 100000 nucleotides, or at least 1000000 nucleotides;
wherein the DNA molecule or composition thereof comprises or consists of a nucleic acid sequence as defined hereinafter:
   (5') [**SEQ ID NO: 40**]ₓ - GoI - Poly(dA/dT) - [**SEQ ID NO: 40**]_{y} (3')
wherein x is an integer number being 0, 1, 2, 3, 4, or 5, preferably 0 or 1; wherein y is a positive integer number being 1, 2, 3, 4, or 5, preferably 1,
preferably wherein the DNA molecule or composition thereof is a circular DNA molecule, a linear DNA molecule, or a concatemer DNA molecule, and
optionally wherein the DNA molecule or composition thereof further comprises one or more component selected from the group consisting of 5' Untranslated Region (5' UTR), 3' Untranslated Region (3' UTR), RNA polymerase promoter region, origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof comprises at least one nucleic acid sequence of **SEQ ID NO: 50;**
wherein the DNA molecule or composition thereof comprises at least 50 nucleotides, at least 100 nucleotides, at least 1000 nucleotides, at least 10000 nucleotides, at least 100000 nucleotides, or at least 1000000 nucleotides;
wherein the DNA molecule or composition thereof comprises or consists of a nucleic acid sequence as defined hereinafter:
   (5') [**SEQ ID NO: 50**]ₓ - GoI - Poly(dA/dT) - [**SEQ ID NO: 50**]_{y} (3')
wherein x is an integer number being 0, 1, 2, 3, 4, or 5, preferably 0 or 1; wherein y is a positive integer number being 1, 2, 3, 4, or 5, preferably 1,
preferably wherein the DNA molecule or composition thereof is a circular DNA molecule, a linear DNA molecule, or a concatemer DNA molecule, and
optionally wherein the DNA molecule or composition thereof further comprises one or more component selected from the group consisting of 5' Untranslated Region (5' UTR), 3' Untranslated Region (3' UTR), RNA polymerase promoter region, origin of replication, antibiotic resistance genes, multiple cloning sites, promoters, selectable markers, reporter genes, fusion tags, termination sequences, origins of transfer sequences, and regulatory elements.

In some embodiments, the DNA molecule or composition thereof comprises at least one nucleic acid sequence of **SEQ ID NO: 53;**
wherein the DNA molecule or composition thereof comprises at least 50 nucleotides, at least 100 nucleotides, at least 1000 nucleotides, at least 10000 nucleotides, at least 100000 nucleotides, or at least 1000000 nucleotides;
wherein the DNA molecule or composition thereof comprises or consists of a nucleic acid sequence as defined hereinafter:
   (5') [**SEQ ID NO: 53**]ₓ - GoI - Poly(dA/dT) - [**SEQ ID NO: 53**]_{y} (3')
wherein x is an integer number being 0, 1, 2, 3, 4, or 5, preferably 0 or 1; wherein y is a positive integer number being 1, 2, 3, 4, or 5, preferably 1,
preferably wherein the DNA molecule or composition thereof is a circular DNA molecule, a linear DNA molecule, or a concatemer DNA molecule, and
optionally wherein the DNA molecule or composition thereof further comprises one or more component selected from the group consisting of 5' Untranslated Region (5' UTR), 3' Untranslated Region (3' UTR), RNA polymerase promoter region, origin of replication, antibiotic resistance genes, multiple cloning sites, promoters, selectable markers, reporter genes, fusion tags, termination sequences, origins of transfer sequences, and regulatory elements.

The present invention further relates to an *in vitro* method of linearizing a circular DNA molecule as described herein, comprising the steps of:
(i) contacting at least one DNA molecule or composition thereof as described herein, with at least one restriction enzyme, thereby obtaining a mixture;
(ii) incubating the mixture obtained at step (i) at a temperature from about 25°C to about 50°C, preferably about 37°C, for a duration from about 10 minutes to about 5 hours, preferably about 1,5 hours, thereby obtaining a linear DNA molecule; and
(iii) optionally, inactivating the at least one restriction enzyme by incubating at a temperature from about 55°C to about 95°C, preferably about 65°C or 80°C, for a duration from about 1 minute to about 1 hour, preferably about 20 minutes;
wherein the at least one restriction enzyme cleaves a restriction site on the at least one nucleic acid sequence of formula (I), or **SEQ ID NO 1.**

The present invention further relates to an *in vitro* method of linearizing a circular DNA molecule comprising at least one sequence of interest as described herein, comprising the steps of:
(i) contacting at least one DNA molecule comprising at least one sequence of interest or composition thereof as described herein, with at least one restriction enzyme, thereby obtaining a mixture;
(ii) incubating the mixture obtained at step (i) at a temperature from about 25°C to about 50°C, preferably about 37°C, for a duration from about 10 minutes to about 5 hours, preferably about 1,5 hours, thereby obtaining a linear DNA molecule; and
(iii) optionally, inactivating the at least one restriction enzyme by incubating at temperature from about 55°C to about 95°C, preferably about 65°C or 80°C, for a duration from about 1 minute to about 1 hour, preferably about 20 minutes;

wherein the at least one restriction enzyme cleaves a restriction site on the at least one nucleic acid sequence of formula (I), or **SEQ ID NO 1,** and
wherein the at least one restriction enzyme does not cleave a restriction site on the at least one sequence of interest.

The present invention further relates to an *in vitro* method of excising at least one sequence of interest from a DNA molecule comprising at least one sequence of interest as described herein, comprising the steps of:
(i) contacting at least one DNA molecule comprising at least one sequence of interest or composition thereof as described herein, with at least one restriction enzyme, thereby obtaining a mixture;
(ii) incubating the mixture obtained at step (i) at a temperature from about 25°C to about 50°C, preferably about 37°C, for a duration from about 10 minutes to about 5 hours, preferably about 1,5 hours, thereby obtaining a linear DNA molecule; and
(iii) optionally, inactivating the at least one restriction enzyme by incubating at temperature from about 55°C to about 95°C, preferably about 65°C or 80°C, for a duration from about 1 minute to about 1 hour, preferably about 20 minutes;

wherein the at least one restriction enzyme cleaves a restriction site on the at least two nucleic acid sequence of formula (I), or **SEQ ID NO 1,** and
wherein the at least one restriction enzyme does not cleave a restriction site on the at least one sequence of interest.

The present invention further relates to an *in vitro* method of linearizing a circular DNA molecule as described herein, comprising the steps of:
(i) providing at least one DNA molecule or composition thereof as described herein,
(ii) contacting the at least one DNA molecule or composition thereof as described herein, provided at step (i) with at least one restriction enzyme, thereby obtaining a mixture;
(iii) incubating the mixture obtained at step (ii) at a temperature from about 25°C to about 50°C, preferably about 37°C, for a duration from about 10 minutes to about 5 hours, preferably about 1,5 hours, thereby obtaining a linear DNA molecule;
(iv) optionally, inactivating the at least one restriction enzyme by incubating at a temperature from about 55°C to about 95°C, preferably about 65°C or 80°C, for a duration from about 1 minute to about 1 hour, preferably about 20 minutes;
   wherein the at least one restriction enzyme cleaves a restriction site on the at least one nucleic acid sequence of formula (I), or **SEQ ID NO 1,** and
(v) optionally, recovering the linearized DNA molecule.

The present invention further relates to an *in vitro* method of linearizing a circular DNA molecule comprising at least one sequence of interest as described herein, comprising the steps of:
(i) providing at least one DNA molecule or composition thereof as described herein,
(ii) contacting at least one DNA molecule comprising at least one sequence of interest or composition thereof as described herein provided at step (i), with at least one restriction enzyme, thereby obtaining a mixture;
(iii) incubating the mixture obtained at step (ii) at a temperature from about 25°C to about 50°C, preferably about 37°C, for a duration from about 10 minutes to about 5 hours, preferably about 1,5 hours, thereby obtaining a linear DNA molecule; and
(iv) optionally, inactivating the at least one restriction enzyme by incubating at temperature from about 55°C to about 95°C, preferably about 65°C or 80°C, for a duration from about 1 minute to about 1 hour, preferably about 20 minutes; and
(v) optionally, recovering the linearized DNA molecule;

wherein the at least one restriction enzyme cleaves a restriction site on the at least one nucleic acid sequence of formula (I), or **SEQ ID NO 1,** and
wherein the at least one restriction enzyme does not cleave a restriction site on the at least one sequence of interest.

The present invention further relates to an *in vitro* method of excising at least one sequence of interest from a DNA molecule comprising at least one sequence of interest as described herein, comprising the steps of:
(i) providing at least one DNA molecule or composition thereof as described herein,
(ii) contacting at least one DNA molecule comprising at least one sequence of interest or composition thereof as described herein provided at step (i), with at least one restriction enzyme, thereby obtaining a mixture;
(iii) incubating the mixture obtained at step (ii) at a temperature from about 25°C to about 50°C, preferably about 37°C, for a duration from about 10 minutes to about 5 hours, preferably about 1,5 hours, thereby obtaining a linear DNA molecule; and
(iv) optionally, inactivating the at least one restriction enzyme by incubating at temperature from about 55°C to about 95°C, preferably about 65°C or 80°C, for a duration from about 1 minute to about 1 hour, preferably about 20 minutes;
   wherein the at least one restriction enzyme cleaves a restriction site on the at least two nucleic acid sequence of formula (I), or **SEQ ID NO 1,** and
(v) optionally, recovering the excised DNA molecule;
wherein the at least one restriction enzyme does not cleave a restriction site on the at least one sequence of interest.

In a preferred embodiment, the DNA molecule comprising at least one sequence of interest as described herein comprises at least two nucleic acid sequence of formula (I), or **SEQ ID NO 1,** flanking the at least one sequence of interest.

In some embodiments, the restriction enzyme is selected from the group consisting of AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PieI, SapI, and SfaNI, preferably selected from the group consisting of BpuEI, BcoDI, BsaI, BmrI, EciI, NmeAIII, BsmaI, Esp3I, BciVI, MmeI, AcuI, EarI, SapI, and BtgZI.

In some embodiments, the at least one restriction enzyme is in a buffer suitable for its activity. Such buffers may comprise, illustratively, one or more of salts, a pH buffering agent, serum albumin, or dithiothreitol. Non-limitative examples of suitable buffers may include rCutsmart^{™} (New England Biolabs cat. n° B6004S), NEB r2.1^{™} (New England Biolabs cat. n° B6002S), FastDigest Buffer (Thermo Fischer Scientific^{™} cat. n° B64), or Multi-core buffer^{™} (Promega cat. n° R9991).

In some embodiments the restrictions enzymes as described herein cleave or cut the DNA molecule as described herein on the same site. In some embodiments, the DNA molecule is cleaved or cut at a site adjacent to the at least one sequence of interest. In a preferred embodiment, the DNA molecule is cleaved or cut at a site adjacent to the poly(dA) or poly(dT) nucleic acid sequence. In some embodiments the plurality of restrictions enzymes as described herein cleaves or cuts the DNA molecule as described herein so that there is no nucleotide in 3' after the poly(dA) or poly(dT) nucleic acid sequence.

The present invention further relates to a method of *in vitro* transcription, comprising the steps of:
(i) contacting at least one DNA molecule or composition thereof as described herein, with at least one RNA polymerase; and
(ii) performing *in vitro* transcription of said DNA molecule, thereby providing at least one RNA molecule, in particular at least one messenger RNA (mRNA).

The DNA molecule or composition thereof may comprise at least one sequence of interest as described herein. The present invention further relates to a method of *in vitro* transcription, comprising the steps of:
(i) contacting at least one DNA molecule comprising at least one sequence of interest or composition thereof as described herein, with at least one RNA polymerase; and
(ii) performing *in vitro* transcription of said DNA molecule, thereby providing at least one RNA molecule, in particular a messenger RNA (mRNA), comprising the nucleic acid sequence of interest.

Withing the scope of the present invention, preferred RNA molecules are messenger RNA (mRNA) and self-amplifying RNA (saRNA). In some embodiments, the mRNA is a self-amplifying RNA (saRNA), or self-amplifying messenger RNA.

In some embodiments, the RNA is not a circRNA.

In a preferred embodiment, the DNA molecule comprising at least one sequence of interest as described herein further comprises a poly(dA) or poly(dT) nucleic acid sequence.

In some embodiments, the DNA molecule comprising at least one sequence of interest as described herein comprises at least two nucleic acid sequences of formula (I), or **SEQ ID NO 1,** flanking the at least one sequence of interest. In some embodiments, the DNA molecule comprising at least one sequence of interest as described herein comprises one nucleic acid sequence of formula (I), or **SEQ ID NO 1,** flanking the at least one sequence of interest in 3', preferably flanking the poly(dA/dT) nucleic acid sequence in 3 '.

In a preferred embodiment, the DNA molecule comprising at least one sequence of interest as described herein comprises, from 5' to 3': optionally a nucleic acid sequence of formula (I), or **SEQ ID NO 1;** the nucleic acid sequence of interest; a poly(dA/dT) nucleic acid sequence; a nucleic acid sequence of formula (I), or **SEQ ID NO 1.**

Suitable RNA polymerases are known in the art and include, illustratively, T7 RNA polymerase or *E. coli* RNA polymerase.

In some embodiments, the at least one DNA molecule comprising at least one sequence of interest or composition thereof further comprises at least one RNA polymerase promoter region.

The nucleic acid sequence of formula (I) or **SEQ ID NO: 1** (included in the DNA molecule or composition thereof according to the invention) comprises at least 3 restriction enzyme sites, therefore contacting the DNA molecule of the invention with a suitable restriction enzyme (*i.e.,* a restriction enzyme that recognizes a restriction site on the nucleic acid sequence of formula (I) or **SEQ ID NO: 1**) results in producing a "cleaved" or "cut" DNA molecule, and in the case the DNA molecule is circular, a "linearized" DNA molecule.

Therefore, in some embodiments, the method *of in vitro* transcription comprises the steps of:
(i) contacting at least one DNA molecule comprising at least one nucleic acid sequence of interest and a poly(dA) or poly(dT) nucleic acid sequence, or composition thereof, as described herein, with at least one restriction enzyme, thereby obtaining a cleaved DNA molecule;
(ii) contacting the cleaved DNA molecule obtained at step (i) with at least one RNA polymerase; and
(iii) performing *in vitro* transcription of said cleaved DNA molecule, thereby providing at least one RNA molecule, in particular at least one messenger RNA (mRNA), for example a self-amplifying messenger RNA (saRNA), comprising the nucleic acid sequence of interest;

wherein the at least one restriction enzyme cleaves a restriction site on the at least one nucleic acid sequence of formula (I), or **SEQ ID NO 1,** and
wherein the at least one restriction enzyme does not cleave a restriction site on the at least one nucleic acid sequence of interest.

In some embodiments, the method *of in vitro* transcription comprises, before step (i), a step of providing at least one DNA molecule comprising at least one sequence of interest and a poly(dA) or poly(dT) nucleic acid sequence, or composition thereof, as described herein. In some embodiments, the method of *in vitro* transcription comprises, before step (i), a step of providing at least one DNA molecule or composition thereof comprising or consisting of a nucleic acid sequence as defined hereinafter:
(5') [first formula (I) sequence]ₓ - GoI - Poly(dA/dT) - [second formula (I) sequence]_{y} (3')
wherein x is an integer number being 0, 1, 2, 3, 4, or 5, preferably 0 or 1; wherein y is a positive integer number being 1, 2, 3, 4, or 5, preferably 1,
wherein the first formula (I) sequence and the second formula (I) sequence may be the same or different.

In some embodiments, the restriction enzyme is selected from the group consisting of AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PieI, SapI, and SfaNI, preferably selected from the group consisting of BpuEI, BcoDI, BsaI, BmrI, EciI, NmeAIII, BsmaI, Esp3I, BciVI, MmeI, AcuI, EarI, SapI, and BtgZI.

In some embodiments, the at least one restriction enzyme is in a buffer suitable for its activity. Such buffers may comprise, illustratively, one or more of salts, a pH buffering agent, serum albumin, or dithiothreitol. Non-limitative examples of suitable buffers may include rCutsmart^{™} (New England Biolabs cat. n° B6004S), NEB r2.1^{™} (New England Biolabs cat. n° B6002S), FastDigest Buffer (Thermo Fischer Scientific^{™} cat. n° B64), or Multi-core buffer^{™} (Promega cat. n° R9991).

In some embodiments the restrictions enzymes as described herein cleave or cut the DNA molecule as described herein on the same site. In some embodiments, the DNA molecule is cleaved or cut at a site adjacent to the at least one sequence of interest. In a preferred embodiment, the DNA molecule is cleaved or cut at a site adjacent to the poly(dA) or poly(dT) nucleic acid sequence. In some embodiments the plurality of restrictions enzymes as described herein cleaves or cuts the DNA molecule as described herein so that there is no nucleotide in 3' after the poly(dA) or poly(dT) nucleic acid sequence.

In some embodiments, the step of contacting at least one DNA molecule comprising at least one sequence of interest or composition thereof as described herein, with at least one restriction enzyme, further comprises incubating at a temperature from about 25°C to about 50°C, preferably about 37°C, for a duration from about 10 minutes to about 5 hours, preferably about 1,5 hours, thereby obtaining a cleaved or cut DNA molecule; and optionally, inactivating the at least one restriction enzyme by incubating at temperature from about 55°C to about 95°C, preferably about 65°C or 80°C, for a duration from about 1 minute to about 1 hour, preferably about 20 minutes. In some embodiment, when the at least one DNA molecule comprising at least one sequence of interest or composition thereof as described herein is a circular DNA molecule, the obtained DNA molecule is a linearized DNA molecule.

In some embodiments, at the step of contacting the at least one DNA molecule with the at least one RNA polymerase, the at least one DNA molecule is also contacted with a suitable buffer and a plurality of ribonucleotide tri-phosphate (rNTP), and optionally at least one reducing agent and/or a pyrophosphatase.

In some embodiments, the step of performing *in vitro* transcription further comprises incubating at a temperature from about 25°C to about 50°C, preferably about 37°C, for a duration from about 1 hour to about 48 hours, preferably from about 10 hours to about 20 hours.

In some embodiments, the step of performing *in vitro* transcription is performed in presence of a suitable buffer and a plurality of ribonucleotide tri-phosphate (rNTP).

In some embodiments, the step of performing *in vitro* transcription is repeated any number of times. In some embodiments, the step of performing *in vitro* transcription is repeated 2 times, 3 times, 4 times, 5 times, 10 times, 20 times, or more. It will be apparent to the person skilled in the art that repeating *in vitro* transcription may yield a higher quantity of mRNA produced.

In some embodiments, the method of *in vitro* transcription further comprises a step of eliminating the remaining DNA, preferably with DNAse. It will be apparent to the person skilled in the art that the step of eliminating the remaining DNA must take place after the step of performing *in vitro* transcription.

In some embodiments, the method *of in vitro* transcription further comprises a step of recovering the obtained mRNA molecule.

In some embodiments, the method of *in vitro* transcription further comprises a step of purifying the obtained mRNA molecule. Non-limitative examples of techniques for mRNA purification include reversed phase purification, ion exchange purification, size exclusion purification, hydrophobic interaction purification, and affinity purification.

In some embodiments, the method *of in vitro* transcription further comprises a step of sequencing the obtained RNA molecule, in particular the mRNA.

The obtained RNA molecule, in particular the mRNA, may be stored in suitable conditions known in the art, e.g., at -20°C or -80°C in a suitable RNAse-free buffer.

The present invention further relates to a method of *in vitro* replication or amplification, comprising the steps of:
(i) contacting at least one DNA molecule or composition thereof as described herein, with at least one DNA polymerase, and
(ii) performing *in vitro* replication of said DNA molecule, thereby providing at least one copy of said DNA molecule.

Suitable DNA polymerases are known in the art and include, illustratively, Phi29 DNA polymerase, Bsu DNA polymerase, Bst DNA polymerase, or Taq DNA polymerase.

In certain embodiments, the method of *in vitro* replication or amplification is a method of isothermal amplification. In certain embodiments, the method of *in vitro* replication or amplification is a method of rolling circle amplification. In certain embodiments, the method of *in vitro* replication or amplification is a method of theta amplification.

In some embodiments, the at least one DNA molecule comprising at least one sequence of interest or composition thereof further comprises at least one origin of replication.

In some embodiments, the method of *in vitro* replication or amplification comprises the steps of:
(i) optionally, contacting at least one DNA molecule comprising at least one sequence of interest or composition thereof as described herein, with at least one restriction enzyme, thereby obtaining a cleaved DNA molecule;
(ii) contacting the at least one DNA molecule comprising at least one sequence of interest or composition thereof as described herein, or the cleaved DNA molecule obtained at step (i), with at least one DNA polymerase; and
(iii) performing *in vitro* replication or amplification on the DNA molecule comprising at least one sequence of interest, or on the cleaved DNA molecule obtained at step (i), thereby providing at least one copy of the DNA molecule comprising the nucleic acid sequence of interest, or a complementary sequence thereof;

wherein the at least one restriction enzyme cleaves a restriction site on the at least one nucleic acid sequence of formula (I), or **SEQ ID NO 1,** and
wherein the at least one restriction enzyme does not cleave a restriction site on the at least one sequence of interest.

In some embodiments, the method of *in vitro* replication or amplification comprises, before step (i), a step of providing at least one DNA molecule comprising at least one sequence of interest, or composition thereof, as described herein.

In some embodiments, the restriction enzyme is selected from the group consisting of AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PieI, SapI, and SfaNI, preferably selected from the group consisting of BpuEI, BcoDI, BsaI, BmrI, EciI, NmeAIII, BsmaI, Esp3I, BciVI, MmeI, AcuI, EarI, SapI, and BtgZI.

In some embodiments, the at least one restriction enzyme is in a buffer suitable for its activity. Such buffers may comprise, illustratively, one or more of salts, a pH buffering agent, serum albumin, or dithiothreitol. Non-limitative examples of suitable buffers may include rCutsmart^{™} (New England Biolabs cat. n° B6004S), NEB r2.1^{™} (New England Biolabs cat. n° B6002S), FastDigest Buffer (Thermo Fischer Scientific^{™} cat. n° B64), or Multi-core buffer^{™} (Promega cat. n° R9991).

In some embodiments the restrictions enzymes as described herein cleave or cut the DNA molecule as described herein on the same site. In some embodiments, the DNA molecule is cleaved or cut at a site adjacent to the at least one sequence of interest. In a preferred embodiment, the DNA molecule is cleaved or cut at a site adjacent to the poly(dA) or poly(dT) nucleic acid sequence. In some embodiments the plurality of restrictions enzymes as described herein cleaves or cuts the DNA molecule as described herein so that there is no nucleotide in 3' after the poly(dA) or poly(dT) nucleic acid sequence.

In some embodiments, the step of contacting at least one DNA molecule comprising at least one sequence of interest or composition thereof as described herein, with at least one restriction enzyme, further comprises incubating at a temperature from about 25°C to about 50°C, preferably about 37°C, for a duration from about 10 minutes to about 5 hours, preferably about 1,5 hours, thereby obtaining a cleaved or cut DNA molecule; and optionally, inactivating the at least one restriction enzyme by incubating at temperature from about 55°C to about 95°C, preferably about 65°C or 80°C, for a duration from about 1 minute to about 1 hour, preferably about 20 minutes. In some embodiment, when the at least one DNA molecule comprising at least one sequence of interest or composition thereof as described herein is a circular DNA molecule, the obtained DNA molecule is a linearized DNA molecule.

In some embodiments, at the step of contacting the at least one DNA molecule with the at least one DNA polymerase, the at least one DNA molecule is also contacted with a suitable buffer, a plurality of deoxyribonucleotide tri-phosphate (dNTP), and optionally one or more primers.

In some embodiments, the step of performing *in vitro* replication or amplification further comprises incubating at a temperature from about 25°C to about 50°C, preferably about 37°C, for a duration from about 1 hour to about 48 hours, preferably from about 10 hours to about 20 hours.

In some embodiments, the step of performing *in vitro* replication or amplification is performed in presence of a suitable buffer, a plurality of deoxyribonucleotide tri-phosphate (dNTP), and optionally one or more primers.

In some embodiments, the step of performing *in vitro* replication or amplification is repeated any number of times. In some embodiments, the step of performing *in vitro* replication or amplification is repeated 2 times, 3 times, 4 times, 5 times, 10 times, 20 times, or more.

In some embodiments, the method *of in vitro* replication or amplification further comprises a step of eliminating contaminating RNA, preferably with RNAse.

In some embodiments, the method of *in vitro* transcription further comprises a step of recovering the obtained DNA molecule.

In some embodiments, the method *of in vitro* replication or amplification further comprises a step of purifying the obtained DNA molecule. Non-limitative examples of techniques for DNA purification include reversed phase purification, ion exchange purification, size exclusion purification, hydrophobic interaction purification, and affinity purification.

In some embodiments, the method *of in vitro* replication or amplification further comprises a step of sequencing the obtained DNA molecule.

The obtained DNA molecule may be stored in suitable conditions known in the art, e.g., at -20°C or -80°C in a suitable buffer or demineralized water.

The obtained DNA molecule may be transfected in a cell, in particular a bacterium.

The present invention further relates to an *in vitro* method of cloning, comprising the steps of:
(i) contacting at least one DNA molecule or composition thereof as described herein, with at least one restriction enzyme, thereby obtaining a cleaved DNA molecule;
(ii) contacting the cleaved DNA molecule obtained at step (i) with at least one nucleic acid sequence of interest, and at least one DNA ligase or the like, and
(iii) inserting said at least one nucleic acid sequence of interest in the cleaved DNA molecule, thereby providing a DNA molecule comprising the nucleic acid sequence of interest; and
(iv) optionally, recovering the obtained DNA molecule;

wherein the at least one restriction enzyme cleaves a restriction site on the at least one nucleic acid sequence of formula (I), or **SEQ ID NO 1,** and
wherein the at least one restriction enzyme does not cleave a restriction site on the at least one sequence of interest.

The present invention further relates to an *in vitro* method of cloning, comprising the steps of:
(i) contacting at least one DNA molecule comprising at least one nucleic acid sequence of formula (I), or **SEQ ID NO 1,** with at least one restriction enzyme, thereby obtaining a cleaved DNA molecule;
(ii) contacting the cleaved DNA molecule obtained at step (i) with at least one nucleic acid sequence of interest, and at least one DNA ligase or the like, and
(iii) inserting said at least one nucleic acid sequence of interest in the cleaved DNA molecule, thereby providing a DNA molecule comprising the nucleic acid sequence of interest; and
(iv) optionally, recovering the obtained DNA molecule;

wherein the at least one restriction enzyme cleaves a restriction site on the at least one nucleic acid sequence of formula (I), or **SEQ ID NO 1,** and
wherein the at least one restriction enzyme does not cleave a restriction site on the at least one sequence of interest.

In some embodiments, the *in vitro* method of cloning comprises, before step (i), a step of providing at least one DNA molecule, or composition thereof, as described herein.

The the *in vitro* method of cloning may be a method of inserting at least one nucleic acid sequence of interest in a DNA molecule comprising at least one nucleic acid sequence of formula (I), or **SEQ ID NO 1.**

In some embodiments, the restriction enzyme is selected from the group consisting of AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PieI, SapI, and SfaNI, preferably selected from the group consisting of BpuEI, BcoDI, BsaI, BmrI, EciI, NmeAIII, BsmaI, Esp3I, BciVI, MmeI, AcuI, EarI, SapI, and BtgZI.

In some embodiments, the at least one restriction enzyme is in a buffer suitable for its activity. Such buffers may comprise, illustratively, one or more of salts, a pH buffering agent, serum albumin, or dithiothreitol. Non-limitative examples of suitable buffers may include rCutsmart^{™} (New England Biolabs cat. n° B6004S), NEB r2.1^{™} (New England Biolabs cat. n° B6002S), FastDigest Buffer (Thermo Fischer Scientific^{™} cat. n° B64), or Multi-core buffer^{™} (Promega cat. n° R9991).

In some embodiments the restrictions enzymes as described herein cleave or cut the DNA molecule as described herein on the same site. In some embodiments, the DNA molecule is cleaved or cut at a site adjacent to the at least one sequence of interest. In a preferred embodiment, the DNA molecule is cleaved or cut at a site adjacent to the poly(dA) or poly(dT) nucleic acid sequence. In some embodiments the plurality of restrictions enzymes as described herein cleaves or cuts the DNA molecule as described herein so that there is no nucleotide in 3' after the poly(dA) or poly(dT) nucleic acid sequence.

In some embodiments, the step of contacting at least one DNA molecule comprising at least one sequence of interest or composition thereof as described herein, with at least one restriction enzyme, further comprises incubating at a temperature from about 25°C to about 50°C, preferably about 37°C, for a duration from about 10 minutes to about 5 hours, preferably about 1,5 hours, thereby obtaining a cleaved or cut DNA molecule; and optionally, inactivating the at least one restriction enzyme by incubating at temperature from about 55°C to about 95°C, preferably about 65°C or 80°C, for a duration from about 1 minute to about 1 hour, preferably about 20 minutes. In some embodiment, when the at least one DNA molecule comprising at least one sequence of interest or composition thereof as described herein is a circular DNA molecule, the obtained DNA molecule is a linearized DNA molecule.

In some embodiments, the obtained DNA molecule comprising the nucleic acid sequence of interest may be amplified or replicated with the method of *in vitro* replication or amplification as described herein, or with any suitable DNA replication or amplification method known in the art. In some embodiments, the obtained DNA molecule comprising the nucleic acid sequence of interest may be transfected in a cell, in particular a bacterium.

The present invention further relates to a vector comprising the DNA molecule or composition thereof as described herein. In some embodiments, the vector is selected from the group consisting of viral vectors, plasmids vectors, fosmids vectors, cosmids vectors, artificial chromosomes vectors, nanoparticles vectors, minicircle DNA vectors, transposase-based vectors, integrase-based vectors, CRISPR-Cas-based vectors, and the like.

The present invention further relates to a recombinant host cell or microorganism or viral particle comprising the DNA molecule or composition thereof, comprising at least one nucleic acid sequence of formula (I), or **SEQ ID NO 1:**
(I) X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆; wherein:
X₁ is G;
X₂ is selected from a group consisting of: A, C or G;
X₃ is selected from a group consisting of: A, G or T;
X₄ is selected from a group consisting of: A, C, G or T;
X₅ is selected from a group consisting of: A, C or T;
X₆ is selected from a group consisting of: A, C, G or T;
X₇ is selected from a group consisting of: C or G;
X₈ is selected from a group consisting of: A or G;
X₉ is selected from a group consisting of: G or T;
X₁₀ is selected from a group consisting of: A, C, G or T;
X₁₁ is selected from a group consisting of: A, C or T;
X₁₂ is selected from a group consisting of: C, G or T;
X₁₃ is C;
X₁₄ is selected from a group consisting of: A, G or T;
X₁₅ is selected from a group consisting of: A or C;
X₁₆ is C;
X₁₇ is T;
X₁₈ is selected from a group consisting of: C, G or T;
X₁₉ is selected from a group consisting of: A or C;
X₂₀ is selected from a group consisting of: A or G;
X₂₁ is selected from a group consisting of: C, G or T;
X₂₂ is selected from a group consisting of: C or T;
X₂₃ is selected from a group consisting of: C or G;
X₂₄ is G;
X₂₅ is selected from a group consisting of: A or G;
X₂₆ is C.

In some embodiments, the recombinant host cell is a prokaryotic cell or a eukaryotic cell. In one embodiment, the recombinant host cell is a prokaryotic cell, preferably selected from the group comprising or consisting of bacteria and archaea, more preferably bacteria. In another embodiment, the recombinant host cell is a eukaryotic cell, preferably selected from the group comprising or consisting of an animal cell, a plant cell, and a fungi cell. In certain embodiments, the fungi cell is a yeast. In certain embodiments, the animal cell is a mammalian cell, preferably a human cell. In some embodiments, the recombinant host cell is a yeast, a bacterium, or a mammalian cell. In some embodiments, the recombinant host cell is a bacterium.

In some embodiments, the microorganism host cell is selected from the group consisting of bacteria, archaea, protozoa, algae, fungi, helminths.

In some embodiments, the viral particle is derived from a virus, or is a part or fragment of a virus, or is an inactivated virus. In some embodiments, the viral particle does not retain replication capabilities from the virus. Non-limitative examples of virus families include *Retroviridae, Adenoviridae, Flaviviridae, Herpesviridae, Hepadnaviridae, Papillomaviridae, Polyomaviridae, Parvoviridae, Arenaviridae, Bornaviridae, Bunyaviridae, Filoviridae* and *Paramyxoviridae* families of viruses, and the like. In some embodiments, the viral particle is selected from the group consisting of adenoviral particle, adeno-associated virus particle, lentiviral particle, integration-deficient lentiviral particle, poxviral particle, herpes simplex virus particle. In some embodiments, the viral particle is a viral vector, preferably an adenoviral vector, adeno-associated virus vector, lentiviral vector, integration-deficient lentiviral vector, poxviral vector, or herpes simplex virus vector.

The present invention further relates to a nucleic acid molecule that encodes the DNA molecule as described herein. In this specific aspect of the invention, the term "encodes" means that the nucleic acid may be retrotranscribed in the DNA molecule as described herein (for example when the nucleic acid molecule is an RNA molecule) or may be complementary to the DNA molecule as described herein (for example when the nucleic acid molecule is a cDNA molecule.

The present invention further relates to a self-amplifying RNA (saRNA), complementary DNA (cDNA) or messenger RNA (mRNA) encoding the DNA molecule as described herein, in particular a saRNA. In some embodiments, the RNA is not a circular RNA (circRNA).

The person skilled in the art can routinely determine the DNA sequences complementary the nucleic acid sequences selected from the group consisting of **SEQ ID NO: 1** to **SEQ ID NO: 57,** and the RNA sequence (e.g. the mRNA molecules) whose retrotranscription would yield nucleic acid sequences selected from the group consisting of **SEQ ID NO: 1** to **SEQ ID NO: 57.**

The present invention further relates to a kit comprising:
- a DNA molecule, or composition thereof, as described herein, or a recombinant host cell or microorganism or viral particle as described herein, or a cDNA or a RNA molecule (in particular a mRNA) as described herein, and
- instructions for use.

In some embodiments, the kit further comprises a restriction enzyme, preferably selected from the group consisting of AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PieI, SapI, and SfaNI, more preferably selected from the group consisting of BpuEI, BcoDI, BsaI, BmrI, EciI, NmeAIII, BsmaI, Esp3I, BciVI, MmeI, AcuI, EarI, SapI, and BtgZI.

In some embodiments, the kit further comprises reagents for *in vitro* transcription, such as a RNA polymerase, rNTP, a buffer, a DNAse, and the like.

In some embodiments, the kit further comprises reagents for *in vitro* replication, such as a DNA polymerase, dNTP, a buffer, a RNAse, and the like.

The present invention further relates to a RNA molecule, in particular a mRNA, obtained by the *in vitro* transcription method as described herein.

The present invention further relates to a pharmaceutical composition comprising a mRNA obtained by the *in vitro* transcription method as described herein.

The present invention further relates to a vaccine composition comprising a RNA molecule, in particular a mRNA, obtained by the *in vitro* transcription method as described herein.

The present invention further relates to the use of a RNA molecule, in particular of a mRNA, obtained by the *in vitro* transcription method as described herein and/or the use of a DNA molecule or composition thereof as described herein, for transfecting a cell or population of cells *in vitro.*

The present invention further relates to a method of transfecting a cell or population of cells *in vitro* with the DNA molecule or composition thereof as described herein. The present invention further relates to a method of transfecting a cell or population of cells *in vitro* with the RNA molecule, in particular the mRNA, obtained by the *in vitro* transcription method as described herein.

The present invention further relates to a RNA molecule, in particular a mRNA, obtained by the *in vitro* transcription method as described herein for use as a medicament.

The present invention further relates to a RNA molecule, in particular a mRNA, obtained by the *in vitro* transcription method as described herein, or pharmaceutical composition comprising thereof, for use for preventing and/or treating a disease selected from the group consisting of genetic diseases, cancer and infectious diseases.

The present invention further relates to method of preventing and/or treating a disease selected from the group consisting of genetic diseases, cancer and infectious diseases, in a subject in need thereof, comprising administering to said subject a therapeutically effective amount of a RNA molecule, in particular a mRNA, obtained by the *in vitro* transcription method as described herein, or pharmaceutical composition comprising thereof.

The present invention further relates to method of vaccinating against a disease selected from the group consisting of infectious diseases and cancer, in a subject in need thereof, comprising administering to said subject a therapeutically effective amount of a RNA molecule, in particular a mRNA, obtained by the *in vitro* transcription method as described herein, or vaccine composition comprising thereof.

The present invention further relates to the use of a RNA molecule, in particular a mRNA, obtained by the *in vitro* transcription method as described herein, or pharmaceutical composition comprising thereof, for the manufacture of a medicament, preferably a vaccine, for preventing and/or treating a disease selected from the group consisting of genetic diseases, cancer and infectious diseases.

The present invention further relates to an isolated nucleic acid sequence comprising the sequence selected from the group consisting **of SEQ ID NO:1** to **SEQ ID NO: 57,** or from the group consisting of **SEQ ID NO:4** to **SEQ ID NO: 57.**

The present invention further relates to an isolated nucleic acid sequence consisting of the sequence selected from the group consisting of **SEQ ID NO:1** to **SEQ ID NO: 57,** or from the group consisting of **SEQ ID NO:4** to **SEQ ID NO:** 57.

### BRIEF DESCRIPTION OF THE DRAWINGS

**Figure 1** is a photograph of a gel electrophoresis showing virtual digestion for the ULP5S.
**Figure 2** is a photograph of a gel electrophoresis showing virtual digestion for the ULP36.
**Figure 3** is a photograph of a gel electrophoresis showing virtual digestion for the ULP46.
**Figure 4** is a photograph of a gel electrophoresis showing virtual digestion for the ULP49.
**Figure 5** is a photograph of a gel electrophoresis showing the digestion of ULPs (without template).
**Figure 6** is a photograph of a gel electrophoresis showing digestion of ULPs (with template).

### EXAMPLES

The present invention is further illustrated by the following examples.

### Example 1:

This example illustrates the design of DNA sequences that contain multiple (from 4 to 5) type IIs restriction enzymes that cut strictly at the end of the polyA tail at the same nucleotide on the template strand. These DNA sequences are herein referred to as "Universal Landing Platform" (ULP).

### Materials and Methods

### Digestion

For each ULP, the digestion has been done following the manufacturer recommendation.

**Table 3: digestion conditions**

| **Component** | **Quantity** |
|---|---|
| Enzyme buffer (10X) | 5 µl |
| ULP DNA | 1 µg |
| Enzyme | 1 µl |
| Nuclease free water | QSP 50 µl |
| Total | 50 µl |

Each digestion has been done for 1H30 at the recommended temperature for each enzyme, and inactivation has been done at 80°C for 20 minutes for all enzymes.

**Table 4: list of enzymes used and conditions**

| **Enzymes** | **ULP** | **SEQ ID NO:** | **Buffer** | **T° of digestion** | **T° of Inactivation** |
|---|---|---|---|---|---|
| BcoDI | ULP5S | 4 | rCutsmart | 37°C | / |
| BsaI | ULP5S | 4 | rCutsmart | 37°C | 80°C |
| | ULP46 | 50 | | | |
| BmrI | ULP5S | 4 | NEB r2.1 | 37°C | / |
| | ULP46 | 50 | | | |
| EciI | ULP5S | 4 | rCutsmart | 37°C | 65°C |
| | ULP46 | 50 | | | |
| NmeAIII | ULP5S | 4 | rCutsmart | 37°C | 65°C |
| | ULP49 | 53 | | | |
| BsmaI | ULP36 | 40 | rCutsmart | 55°C | 65°C |
| Esp3I | ULP36 | 40 | rCutsmart | 37°C | 65°C |
| BciVI | ULP36 | 40 | rCutsmart | 37°C | 80°C |
| MmeI | ULP36 | 40 | rCutsmart | 37°C | 65°C |
| AcuI | ULP46 | 50 | rCutsmart | 37°C | 65°C |
| EarI | ULP49 | 53 | rCutsmart | 37°C | 65°C |
| SapI | ULP49 | 53 | rCutsmart | 37°C | 65°C |
| BtgZI | ULP49 | 53 | rCutsmart | 60°C | 80°C |

### Agarose gel electrophoresis

Once the digestion is done it revealed on agarose gel electrophoresis. 5 µl of digestion product + 1µl of purple dye are deposited on the gel. The gel is done at 1% agarose gel with 1X TAE. The gel is then put to migrate at 110 mV for 1h. After the migration, the gel is put for 45 min to 1 h on a slowly agitating bath of 100 ml of TAE 1X with 7 µl of midori green advance to taint the sample. The gel is then put on a washing bath (5-10 minutes) of TAE 1X. The gel is then revealed.

### DNA purification

Once the digestion has been put on the agarose gel, what is left of the digestion is purified using PCR clean up kit from Qiagen and using the protocol given with the purification kit. This purification will allow to have clean DNA for further use.

### Results

### Digestion

The vector that has been used to test the ULPs is the same for all USPs. For each plasmid of the ULP, a virtual digestion for each enzyme to be used was done to verify the digestion profile.

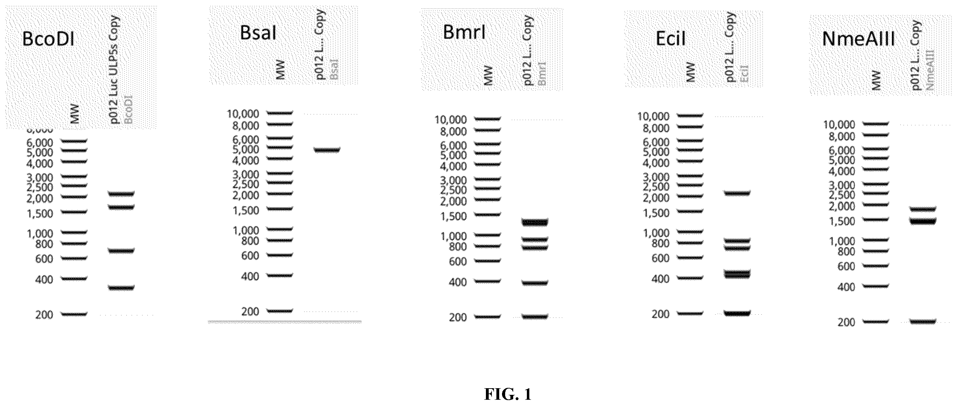

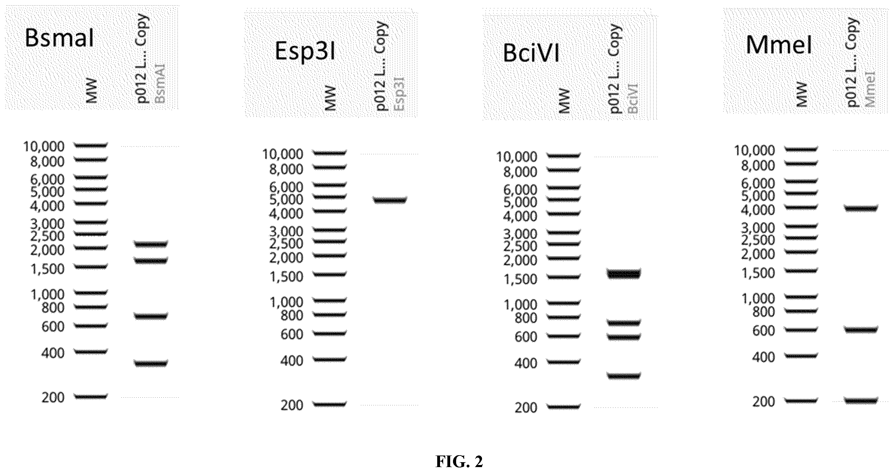

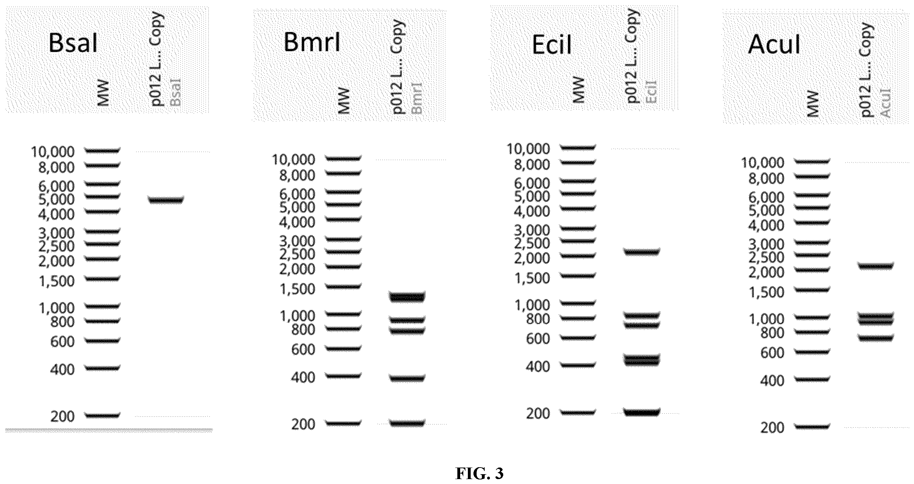

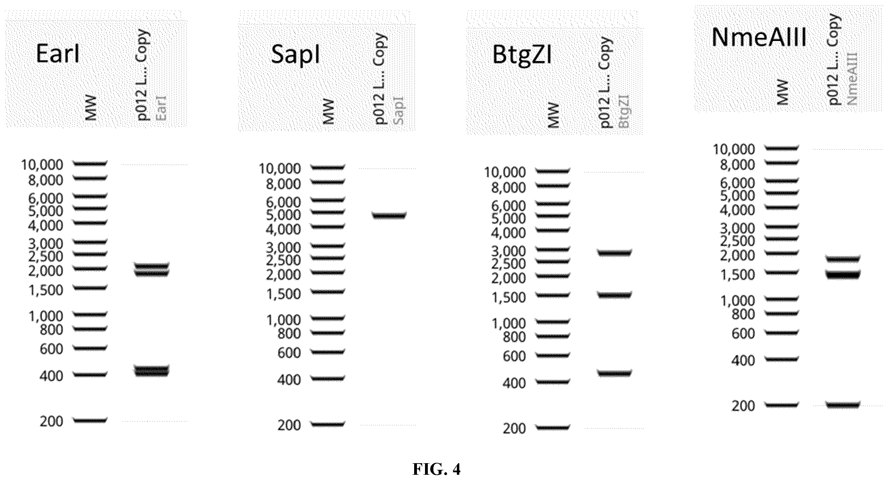

Results for ULP5S (**SEQ ID NO: 4**) are shown on **Figure 1****.** Results for ULP36 (**SEQ ID NO: 40**) are shown on **Figure 2****.** Results for ULP46 (**SEQ ID NO: 50**) are shown on **Figure 3****.** Results for ULP49 (**SEQ ID NO: 53**) are shown on **Figure 4****.**

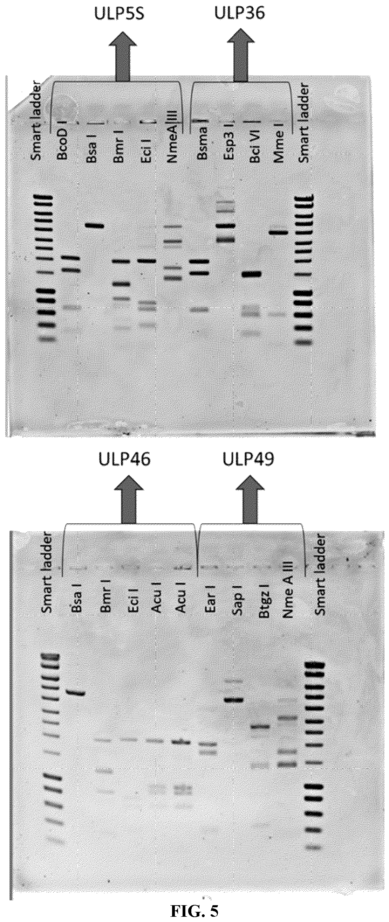

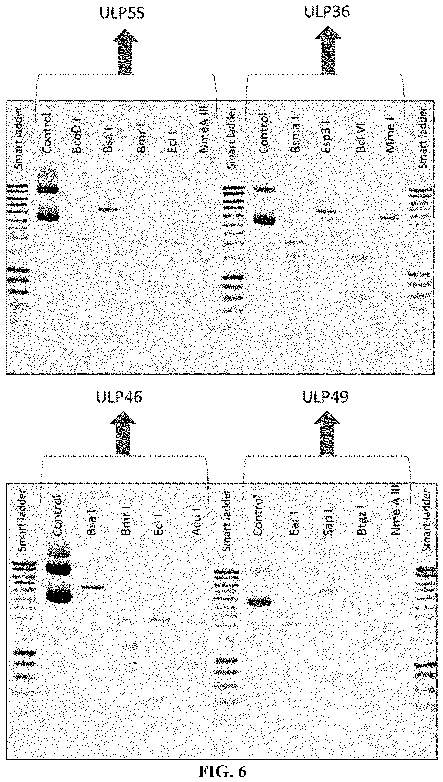

After digestion and revelation as explained in Materials and Methods hereinabove, the results shown on **Figure 5** were obtained. A second gel was done after the purification of the digestion and the plasmid not digested was added (see **Figure 6**).

With the four gels and comparing with the virtual digestions, it can be seen that for each ULP there is at least one enzyme that only cut once on the plasmid at the end of the polyA tail, and therefore they can be used as expected.

BmrI is the only enzyme to have a different buffer and it is a less common enzyme. To simplify the design of the experiment, BmrI went through the denaturation step at 80°C even if not needed (see Table 3), this could explain why the digestion profile is different.

### Conclusion

53 sequences with multiple restriction enzymes sites that cut at the end of any GOI were created, and 4 of those sequences were tested herein. The results show that for these 4 sequences there is at least one enzyme that does not cut in the GOI and can be used for *in vitro* transcription (IVT). This demonstrates that ULP can be used for mRNA production through IVT.

## Claims

1. A deoxyribonucleic acid (DNA) molecule, or composition thereof, comprising at least one nucleic acid sequence of formula (I), or **SEQ ID NO 1:**
(I) **X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆**; wherein:
X₁ is G;
X₂ is selected from a group consisting of: A, C or G;
X₃ is selected from a group consisting of: A, G or T;
X₄ is selected from a group consisting of: A, C, G or T;
X₅ is selected from a group consisting of: A, C or T;
X₆ is selected from a group consisting of: A, C, G or T;
X₇ is selected from a group consisting of: C or G;
X₈ is selected from a group consisting of: A or G;
X₉ is selected from a group consisting of: G or T;
X₁₀ is selected from a group consisting of: A, C, G or T;
X₁₁ is selected from a group consisting of: A, C or T;
X₁₂ is selected from a group consisting of: C, G or T;
X13 is C;
X₁₄ is selected from a group consisting of: A, G or T;
X₁₅ is selected from a group consisting of: A or C;
X₁₆ is C;
X₁₇ is T;
X₁₈ is selected from a group consisting of: C, G or T;
X₁₉ is selected from a group consisting of: A or C;
X₂₀ is selected from a group consisting of: A or G;
X₂₁ is selected from a group consisting of: C, G or T;
X₂₂ is selected from a group consisting of: C or T;
X₂₃ is selected from a group consisting of: C or G;
X24 is G;
X₂₅ is selected from a group consisting of: A or G;
X26 is C.

2. The DNA molecule or composition according to claim **1,** which comprises GVDNHNSRKNHBCDMCTBMRBYSGRC (**SEQ ID NO: 1**);
R being A or G; Y being C or T; M being A or C; K being G or T; S being C or G; W being A or T; B being C, G or T; D being A, G or T; H being A, C or T; V being A, C or G; N being A, C, G or T;
wherein the DNA molecule comprises at least 50 nucleotides.

3. The DNA molecule or composition thereof, according to claim **1** or **2,** which comprises a plurality of restriction enzyme sites selected from type II restriction enzyme sites, preferably type IIC, type IIS and/or type IIP restriction enzyme sites, more preferably selected from the group consisting of: AcuI, AjuI, AlwI, BaeI, BbsI, BbvI, BccI, BceAI, BcgI, BciVI, BcoDI, BfuAI, BmrI, BpmI, BpuEI, BsaI, BsaXI, BseRI, BsgI, BsmAI, BsmFI, BsmI, BspCNI, BspMI, BspQI, BsrDI, BsrI, BtgZI, BtsCI, CspCI, EarI, EciI, Esp3I, FauI, FokI, HgaI, HphI, HpyAV, MboII, MlyI, MmeI, MnlI, NmeAIII, PaqCI, PieI, SapI, and SfaNI restriction sites.

4. The DNA molecule or composition thereof, according to any one of claims **1** to **3,** which comprises a plurality of restriction enzyme sites selected from the group consisting of: BmrI, BpuEI, BsaI, and EciI restriction sites.

5. The DNA molecule or composition thereof, according to claim **3** or **4,** wherein the restriction enzymes corresponding to said plurality of restriction enzyme sites cleave the DNA molecule on the same locus.

6. The DNA molecule or composition thereof, according to any one of claims **1** to **5,** which comprises at least one nucleic acid sequence selected from the group consisting of: **SEQ ID NO: 2** or **SEQ ID NO: 3.**

7. The DNA molecule or composition thereof, according to any one of claims **1** to **5,** which comprises at least one nucleic acid sequence selected from the group consisting of: **SEQ ID NO: 5** to **SEQ ID NO: 57.**

8. The DNA molecule or composition thereof, according to any one of claims **1** to **5,** which comprises at least one nucleic acid sequence **SEQ ID NO: 4.**

9. The DNA molecule or composition thereof, according to any one of claims **1** to **8,** which comprises at least two nucleic acid sequences of formula (I), or **SEQ ID NO: 1,** which may be the same or different.

10. The DNA molecule, or composition thereof, according to any one of claims **1** to **9,** which comprises a poly(dA/dT) nucleic acid sequence; wherein the poly(dA/dT) nucleic acid sequence is **characterized by** a nucleic acid length from 10 nucleotides to 150 nucleotides.

11. The DNA molecule, or composition thereof, according to any one of claims **1** to **10,** further comprising:
- at least one 5' Untranslated Region (5' UTR) and at least one 3' Untranslated Region (3' UTR);
- at least one nucleic acid sequence of interest; and
- a poly(dA/dT) nucleic acid sequence.

12. The DNA molecule, or composition thereof, according to claim **11,** wherein at least one nucleic acid sequence of formula (I), or **SEQ ID NO: 1,** is localized in 5' of said at least one nucleic acid sequence of interest, and at least one nucleic acid sequence of formula (I), or **SEQ ID NO: 1,** is localized in 3' of said poly(dA/dT) nucleic acid sequence.

13. The DNA molecule, or composition thereof, according to any one of claims **1** to **12,** wherein said DNA molecule is a circular DNA molecule, a linear DNA molecule, or a concatemer DNA molecule.

14. A method *of in vitro* transcription, comprising the steps of:
(i) contacting at least one DNA molecule, or composition thereof, according to any one of claims **1** to **13** with at least one RNA polymerase, and
(ii) performing *in vitro* transcription of said DNA molecule, thereby providing at least one RNA molecule, in particular at least one messenger RNA (mRNA).

15. A recombinant host cell or microorganism or viral particle comprising the DNA molecule, or composition thereof, according to any one of claims **1** to **13.**
